Europäisches Patentamt

European Patent Office

Office européen des brevets

(19)

(11) Veröffentlichungsnummer: **0 113 719 B1**

## (12) EUROPÄISCHE PATENTSCHRIFT

(45) Veröffentlichungstag der Patentschrift: **11.03.92**

(51) Int. Cl.⁵: **C12M 1/02**

(21) Anmeldenummer: **82903143.4**

(22) Anmeldetag: **09.10.82**

(86) Internationale Anmeldenummer:
**PCT/EP82/00223**

(87) Internationale Veröffentlichungsnummer:
**WO 84/00378 (02.02.84 84/03)**

(54) **VORRICHTUNG ZUR ERZEUGUNG VON BIOGAS.**

(30) Priorität: **16.07.82 DE 3226698**
**08.09.82 DE 3233367**

(43) Veröffentlichungstag der Anmeldung:
**25.07.84 Patentblatt 84/30**

(45) Bekanntmachung des Hinweises auf die
Patenterteilung:
**11.03.92 Patentblatt 92/11**

(84) Benannte Vertragsstaaten:
**AT BE CH DE FR GB LI LU NL SE**

(56) Entgegenhaltungen:
**FR-A- 1 004 626**
**FR-A- 2 455 847**
**FR-A- 2 480 305**
**FR-A- 2 493 337**
**GB-A- 2 037 731**

(73) Patentinhaber: **Probst, Josef**
**Köckstrasse 9**
**W-8360 Deggendorf(DE)**

(72) Erfinder: **Probst, Josef**
**Köckstrasse 9**
**W-8360 Deggendorf(DE)**

(74) Vertreter: **Gustorf, Gerhard, Dipl.-Ing.**
**Patentanwalt Dipl.-Ing. Gerhard Gustorf**
**Bachstrasse 6 A**
**W-8300 Landshut(DE)**

Rank Xerox (UK) Business Services

**Beschreibung**

Die Erfindung betrifft eine Vorrichtung zur Erzeugung von Biogas aus organischem Substrat mit einer dieses aufnehmenden, eine waagrechte Achse aufweisenden Reaktortrommel mit Rührwerk, die an einer ersten Stirnseite (Beschickungswand) eine Zuführeinrichtung für das frische Substrat und eine Abführeinrichtung für das ausgefaulte Substrat aufweist und mit einer Entnahmeeinrichtung für das erzeugte Biogas sowie mit einer Heizung zur Zuführung der Prozeßwärme ausgerüstet ist.

In der europäischen Patentanmeldung 51 082 ist eine derartige Vorrichtung beschrieben und dargestellt. Bei dieser ist die an ihren beiden Stirnseiten drehbar gelagerte Reaktortrommel in einem allseits geschlossenen Becken untergebracht, das mit Heißwasser für die Zuführung der Prozeßwärme gefüllt ist. Die Zuführeinrichtung für das frische Substrat besteht aus einem in die Reaktortrommel mündenden Rohr, das kurz nach dem Eintritt in die Trommel parallel zur Beschickungswand nach unten abgebogen ist. Von diesem Rohr zweigt ein weiteres Rohr ab, über welches das ausgefaulte Substrat mit Hilfe einer Handpumpe abgeführt werden kann. Das Rührwerk wird durch Mischarme gebildet, die etwa diagonal in der Reaktortrommel angeordnet sind und auf einer festen Achse sitzen.

Abgesehen davon, daß diese bekannte Vorrichtung ein schweres Becken zur Aufnahme und Lagerung der Reaktortrommel benötigt, dessen Baugröße die Außenabmessungen der Reaktortrommel nicht unerheblich überschreitet, wird das frisch zugeführte Substrat während der Gärung ständig mit bereits ausgefaultem Substrat durchmischt, so daß eine optimale Ausnutzung des Substrates nicht möglich ist.

In einer aus der FR-A 24 93 337 bekannten Vorrichtung dient eine in einem Rohr angeordnete Schnecke zur Einspeisung frischen Gärmaterials bis zur Rückwand der Trommel. Es kann jedoch nicht verhindert werden, daß zumindest bei hohem Wassergehalt und dementsprechend dünnflüssigem Reaktorinhalt zonenweise eine Vermengung stattfindet, weil keine Einbauten zur Unterteilung des Reaktorvolumens vorgesehen sind.

Bei der Vorrichtung gemäß GB-A 20 37 731 wird der Gärraum durch koaxiale, gelochte Scheiben unterteilt, die jedoch eher eine Vermengung als eine Abtrennung bewirken.

Der Erfindung liegt die Aufgabe zugrunde, bei einer Vorrichtung der angegebenen Gattung das organische Substrat unter Verbesserung der Energiebilanz so durch die Trommel zu führen, daß eine Vermischung von frischem und an- oder ausgefaultem Substrat weitestgehend verhindert wird.

Bei der gattungsgemäßen Vorrichtung wird diese Aufgabe erfindungsgemäß dadurch gelöst, daß für die Zuführung des frischen Substrates ein mit einem Einfüllbehälter an der Beschickungswand verbundenes Zuführrohr die Reaktortrommel koaxial bis nahe an die gegenüberliegende, zweite Stirnwand durchquert, wo sein Auslauf in die Reaktortrommel mündet, daß um das Zuführrohr herum eine der Länge der Reaktortrommel entsprechende Förderschnecke angeordnet ist, deren Abgabeseite an der Beschickungswand zu einem Abgaberohr führt, und daß die Wand der Reaktortrommel mit der Heizung zur Zuführung der Prozeßwärme ausgerüstet ist.

Bei einer in dieser Weise ausgebildeten Vorrichtung ist sichergestellt, daß während des Prozesses keine unkontrollierte Mischung des frischen Substrates mit dem bereits angefaulten Substrat stattfindet. Vielmehr durchläuft das frische Substrat vor dem Eintritt in die Gärzone der Reaktortrommel das Zuführrohr über die gesamte Trommellänge, bis es an der zweiten Stirnwand in die Förderschnecke gerät und von dieser kontinuierlich zurück zur Beschickungswand transportiert wird, wo es als ausgefaultes Substrat durch das Abgaberohr abgezogen wird. Dabei wird der weitere Vorteil erzielt, daß das frische Substrat, welches das Zuführrohr durchläuft, durch die Wärme, die das bereits in Gärung befindliche Substrat abgibt, im Gegenstrom vorgewärmt wird. Dementsprechend kann die Heizung in der Reaktortrommel optimal ausgelegt werden, wobei hinzukommt, daß gegenüber dem eingangs erläuterten Stand der Technik nur die unmittelbar an den Gärungsraum angrenzende Trommelwand erwärmt werden muß, nicht jedoch ein wesentlich größeres Wasservolumen.

In Weiterbildung der Erfindung ist vorgesehen, daß die Förderschnecke wenigstens auf dem größten Teil ihrer Längserstreckung im Kernbereich einen Abstand zum Zuführrohr aufweist derart, daß in Axialrichtung gesehen eine etwa kreisförmige Durchtrittsöffnung zwischen der Förderschnecke und dem Zuführrohr frei bleibt. Aufgrund dieser Maßnahme wird die gute Durchmischung des Substrates und insbesondere der Sinkschicht und der Schwimmdecke gewährleistet. In vorteilhafter Weise ist dabei die Durchtrittsöffnung im Längsschnitt durch eine gedachte Kegelfläche begrenzt, deren Kegelspitze in der Nähe der Beschickungswand liegt. Damit wird erreicht, daß die Durchmischung im eigentlichen Gärbereich sehr intensiv ist, während auf dem restlichen Teil der Trommellänge vor der Beschickungswand im wesentlichen nur noch eine Förderung des bereits ausgefaulten Substrates zu dem Abgaberohr stattfindet.

Für eine gute Durchmischung ist es weiterhin von Vorteil, wenn die Steigung der Förderschnecke zur zweiten Stirnwand hin zunimmt. Damit wird erreicht, daß das frische Substrat besonders zu Beginn der Gärung und nach dem Verlassen des Auslaufes des Zuführrohres sehr intensiv durchmischt wird, weil dort die Angriffsfläche der Föderschnecke größer ist als im übrigen Längenbereich der Reaktortrommel. Trotzdem erfolgt die Durchmischung so schonend, daß die eingesetzten Mikroorganismen mechanisch nicht beeinflußt werden.

Für einen optimalen Ablauf des Gärprozesses ist es besonders vorteilhaft, wenn zur Relativdrehung zwischen Reaktortrommel und Förderschnecke ein in beiden Drehrichtungen steuerbarer Drehantrieb vorgesehen ist.

Dabei ist vorgesehen, daß der Drehantrieb mit einer Regeleinrichtung verbunden ist, der in die Reaktortrommel eingesetzte Meßsonden zum Erfassen der Prozeßdaten zugeordnet sind.

Aufgrund der durch die Meßsonden erfaßten Prozeßdaten (Temperatur, pH-Wert etc.) kann die Drehung der Reaktortrommel bzw. der Förderschnecke geregelt und damit die Vorrichtung vollständig automatisiert werden. Mit Hilfe der Meßsonden wird nämlich festgestellt, ob das ausgefaulte Substrat, das am Abgaberohr abgezogen wird, vollständig oder nur teilweise ausgebeutet ist, ob das frische Substrat am Auslauf des Zuführrohres die optimale Temperatur hat und dergleichen. Sollten beispielsweise die Prozeßdaten am Abgaberohr ergeben, daß das abgezogene Substrat noch nicht vollständig ausgenutzt ist, so wird über die Regeleinrichtung der Drehantrieb in entgegengesetzter Richtung gesteuert, wodurch das gerade abgezogene Substrat wieder in die Trommel zurückgeholt wird; die Förderschnecke hat dabei nicht nur die Funktion eines Rührwerkes, sondern auch einer Fördereinrichtung, die in Achsrichtung der Trommel wirkt.

Wenn die Reaktortrommel stillstehend ausgebildet ist, kann der Drehantrieb für die Förderschnecke im in Längsrichtung mittleren Bereich der Reaktortrommel befestigt sein. Der Drehantrieb kann bei dieser Lösung in einer gegen die Reaktortrommel abgedichteten Motorkammer angeordnet sein, aus der die Antriebswelle mit einem daran befestigten Antriebsrad in einen Gasraum an der Oberseite der Reaktortrommel ragt. Das Antriebsrad kann dabei als Ritzel ausgebildet sein und mit einem formschlüssig mit dem Schneckenumfang verbundenen Zahnriemen kämmen.

Bei dieser Anordnung bietet sich die Möglichkeit, sowohl die Motorkammer als auch den Gasraum in einer Einstiegsluke an der Oberseite der Reaktortrommel unterzubringen.

Bei der beschriebenen Anordnung bietet sich die Möglichkeit, aus dem Gasraum ein Entnahmerohr zum Abziehen des erzeugten Biogases herauszuführen.

Durch die stillstehende Reaktortrommel und die rotierende Förderschnecke werden nicht nur der Drehantrieb vereinfacht, sondern auch die Verbindung zwischen der Reaktortrommel und der Abführeinrichtung für das ausgefaulte Substrat sowie der Entnahmeeinrichtung für das erzeugte Biogas, da zwischen diesen und der Reaktortrommel keine gleitende Abdichtung erforderlich ist.

In Weiterbildung der Erfindung ist vorgesehen, daß aus der Beschickungswand der Reaktortrommel das Abgaberohr für das ausgefaulte Substrat herausführt, das über eine Kurzschluß leitung mit der Einlaßseite des Zuführrohres verbunden ist, wobei an der Mündung des Zuführrohres in die Kurzschlußleitung ein Dreiweghahn vorgesehen ist.

Dieser Dreiweghahn ist durch eine Regeleinrichtung steuerbar, der Meßsonden zum Erfassen der Prozeßdaten im Zuführbereich des frischen Susbstrates zugeordnet sind.

Wenn bei dieser Ausbildung die Meßsonden feststellen, daß das zugeführte Substrat bereits teilweise oder vollkommen verdorben ist, kann durch die Regeleinrichtung der Dreiweghahn so umgeschaltet werden, daß dieses Substrat von der Einlaßseite des Zuführrohres sofort in die Kurzschlußleitung und von dieser in das Abgaberohr abgeleitet wird, so daß es nicht in die Reaktortrommel gelangt.

Es ist möglich, die drehbare Förderschnecke durch Gleitlager an der zylindrischen Innenwand der Reaktortrommel zu lagern, wobei jedes Gleitlager aus einem am Außenumfang der Förderschnecke befestigten Innenring und aus einem an der Innenwand der Reaktortrommel drehbar abgestützten Außenring besteht, der zu seiner axialen Führung mit dem Innenring in Eingriff ist.

Derartige Gleitlager können je nach der Länge der Förderschnecke und der auftretenden Durchbiegung an mehreren Stellen in Längsrichtung der Reaktortrommel vorgesehen sein. Sowohl der Innenring als auch der Außenring bestehen in vorteilhafter Weise aus verschleißfestem und reibungsarmem Kunststoff, wobei der schwimmend an der Innenwand der Reaktortrommel gelagerte Außenring die Leichtgängigkeit der Schneckenrotation nochmals verbessert.

Nach einem weiteren Merkmal der Erfindung weist die Förderschnecke Durchbrüche für den axialen Durchtritt von Biogas und Substrat auf.

Dabei ist es vorteilhaft, wenn auf jedem Schneckengang nur ein Durchbruch im radial äußeren Bereich vorgesehen ist, alle Durchbrüche in axialer Richtung miteinander fluchten und der Drehantrieb über einen Steuerschalter abschaltbar ist, so daß die Durchbrüche bei Abschaltung der Relativdrehung zwischen Reaktortrommel und Förderschnecke in deren Scheitelbereich liegen.

Im Stillstand der Vorrichtung kann damit das Gas in der immer oben befindlichen Gasblase frei durch die Durchbrüche durchtreten, so daß eine unerwünschte Gaskomprimierung verhindert wird.

In Axialrichtung verlaufende, profilierte Durchmischungsstangen, die an den Schneckengängen der Förderschnecke im radial äußeren Bereich befestigt sind, verhindern die Bildung einer Schwimmdecke und fördern mögliche Sinkschichten nach oben. Auf diese Weise wird ein die Gärung verzögernder Faktor bakterienschonend beseitigt.

Eine weitere Verbesserung des Prozeßablaufes kann dadurch erreicht werden, daß an der Förderschnecke Mischerschaufeln zum Durchmischen des Substrates in beiden Drehrichtungen der Förderschnecke befestigt sind.

Bei einer weiteren Ausführungsform der Erfindung weist die zweite Stirnwand der Reaktortrommel eine Ersatz-Zuführeinrichtung für das frische Substrat auf. Dadurch ist die Möglichkeit gegeben, das frische Substrat an der Seite der Reaktortrommel zuzuführen, die der Abführeinrichtung gegenüberliegt, sofern dies die örtlichen Einbauverhältnisse erfordern.

Reparatur- und Wartungsarbeiten können dadurch erleichtert werden, daß die zweite Stirnwand abnehmbar an der Reaktortrommel befestigt ist.

In Weiterbildung der Erfindung ist die Reaktortrommel an ihrer Beschickungswand mit einer Rücklaufleitung, die etwa in Höhe des Füllpegels aus der Reaktortrommel herausführt, mit dem als Vorgärkammer ausgebildeten Einfüllbehälter verbunden, von dem aus das frische Substrat in das Zuführrohr gelangt, wobei der Querschnitt der Rücklaufleitung wesentlich kleiner ist als derjenige des Abgaberohres.

Über diese Rücklaufleitung kann das noch frische Substrat in der Vorgärkammer geimpft werden, so daß der Gärprozeß früher einsetzt. Dieser Impfvorgang erfolgt durch den bei der Entleerungsdrehung der Förderschnecke ausgeübten Druck zwangsweise.

Es hat sich als besonders vorteilhaft erwiesen, wenn der Einfüllbehälter durch ein Trennelement in eine obere Vorgärkammer und einen unteren Warmwasserspeicher unterteilt ist.

Durch diesen Warmwasserspeicher wird das frische Substrat in der Vorgärkammer bereits vorgewärmt, wobei der Warmwasserspeicher die Prozeßenergie für die Reaktortrommel bereithält.

Eine weitere Verbesserung wird dadurch erzielt, daß das Trennelement ein von außen zugänglicher, horizontaler Aufnahmeschacht für eine Substratpumpe ist, deren Ausgang mit dem Zuführrohr verbunden ist und in deren Eingang ein die Vorgärkammer nach unten begrenzender, nicht isolierter Trichterboden mündet.

Nach einem weiteren Merkmal der Erfindung besteht die in die Trommelwand integrierte Heizung aus Flächenwärmetauschern, die sich in Achsrichtung der Reaktortrommel erstrecken und in Umfangsrichtung gleichmäßig voneinander beabstandet sind.

Die Anordnung kann dabei so getroffen werden, daß jeder Flächenwärmetauscher für das Wärmeübertragungsmedium ein Vorlaufrohr und ein Rücklaufrohr enthält, daß in der Beschickungswand die Vorlaufrohre in wenigstens eine Vorlaufkammer und die Rücklaufrohre in eine Rücklaufkammer münden und daß die Vorlaufkammer und die Rücklaufkammer mit dem Warmwasserspeicher verbunden sind.

Auf diese Weise wird das Substrat in der Reaktortrommel sehr gleichmäßig erwärmt, was für einen einwandfreien Ablauf des Gärungsprozesses erforderlich ist; die in das Substrat eingegebenen Mikroorganismen (Bakterien, Pilze oder andere Zell-kulturen) benötigen nämlich für einen einwandfreien Ablauf des Gärprozesses eine gleichmäßige Temperaturverteilung.

Eine noch gleichmäßigere Wärmeverteilung der zu beheizenden Reaktorinnenfläche wird dadurch erreicht, daß zwei ringförmige, konzentrische Vorlaufkammern vorgesehen sind, in die das Warmwasser im Gegenstrom läuft und von denen jede mit den Vorlaufrohren jedes zweiten Flächenwärmetauschers verbunden ist.

Für eine möglichst maximale Ausnützung der Wärmeenergie weist die Wand des Abgaberohres Wärmetauschelemente zur Aufnahme der Restwärme des ausgefaulten Substrates für die Wärmezufuhr zum Warmwasserspeicher und/oder zur Vorgärkammer auf.

Für die Wärmezuführ zum Warmwasserspeicher und/oder zur Vorgärkammer können Wärmepumpen vorgesehen sein.

Statt des stillstehenden Reaktors und der drehbar angetriebenen Förderschnecke besteht auch die Möglichkeit, die nicht drehbare Förderschnecke sowohl an der zylindrischen Innenwand der drehbaren Reaktortrommel als auch an dem Zuführrohr zu befestigen.

Bei dieser Lösung läßt sich das Abgaberohr zentrisch in der Beschickungswand lagern, wobei das Zuführrohr konzentrisch im Abgaberohr verläuft.

Diese Ausgestaltung im Bereich der Beschickungswand ist besonders kompakt.

Nach einem weiteren Merkmal dieser Variante der Erfindung münden das Zuführrohr und das Abgaberohr in einen Vorsatzbehälter, der durch eine Trennwand in den mit dem Zuführrohr verbundenen, als Vorgärkammer ausgebildeten Einfüllbehälter und in eine mit dem Abgaberohr verbundene Auslaufkammer unterteilt ist.

Dabei ist es vorteilhaft, wenn in die Wand der Vorgärkammer Wärmetauschelemente zur Vorwärmung des frischen Substrates eingebaut sind; die Vorwärmung kann dabei mittels Warmwasser erfolgen, welches durch die von der Reaktortrommel abgegebene Prozeßwärme erhitzt wird.

In analoger Weise können in die Wand der Auslaufkammer Wärmetauschelemente zur Aufnahme der Restwärme des ausgefaulten Substrates eingebaut sein. Diese Restwärme kann für die zuvor erwähnte Vorwärmung des frischen Substrates verwendet werden.

Weitere Merkmale und Vorteile der Erfindung ergeben sich aus den Unteransprüchen und aus der folgenden Beschreibung von Ausführungsbeispielen, die in der Zeichnung dargestellt sind.

Es zeigen:

| | |
|---|---|
| Figur 1 | einen Längsschnitt durch eine Vorrichtung gemäß der Erfindung, |
| Figur 2 | eine perspektivische, teilweise aufgeschnittene Ansicht der Vorrichtung, |
| Figur 3 | eine vergrößerte Schnittdarstellung des Vorsatzbehälters und der Beschickungswand, |
| Figur 4 | einen Querschnitt in der Ebene IV - IV der Figur 3, |
| Figur 5 | eine Draufsicht des Vorsatzbehälters ohne Abdeckung, |
| Figur 6 | eine vergrößerte Schnittdarstellung der Reaktortrommel in der Ebene VI - VI der Figur 1, |
| Figur 7 | einen vergrößerten Teilschnitt der Reaktortrommel im Bereich der zweiten Stirnwand, |
| Figur 8 | eine teilweise geschnittene Darstellung der Reaktortrommel in Richtung des Pfeiles VIII in Figur 1, |
| Figur 9 | einen Längsschnitt durch eine zweite Ausführungsform, |
| Figur 10 | einen Ausschnitt aus Figur 9 in vergrößertem Maßstab, |
| Figur 11 | einen Schnitt in der Ebene XI - XI der Figur 10, |
| Figur 12 | einen Schnitt in der Ebene XII - XII der Figur 10, |
| Figur 13 | einen Querschnitt in der Ebene XIII - XIII der Figur 9, |
| Figur 14 | einen vergrößerten Ausschnitt im Bereich der Kurzschlußleitung in einer ersten Betriebsstellung des Dreiweghahnes, |
| Figur 15 | eine der Figur 14 entsprechende Darstellung in einer zweiten Betriebsstellung des Dreiweghahnes, |
| Figur 16 | eine Ausschnittvergrößerung im Bereich XVI der Figur 10, |
| Figur 17 | eine Draufsicht in Richtung des Pfeiles XVII der Figur 9 bei abgenommenem Deckel, |
| Figur 18 | eine Ansicht in Richtung des Pfeiles XVIII der Figur 11 und |
| Figur 19 | im Längsschnitt eine Teildarstellung in vergrössertem Maßstab eines die Förderschnecke an der Reaktortrommel-Innenwand abstützenden Gleitlagers. |

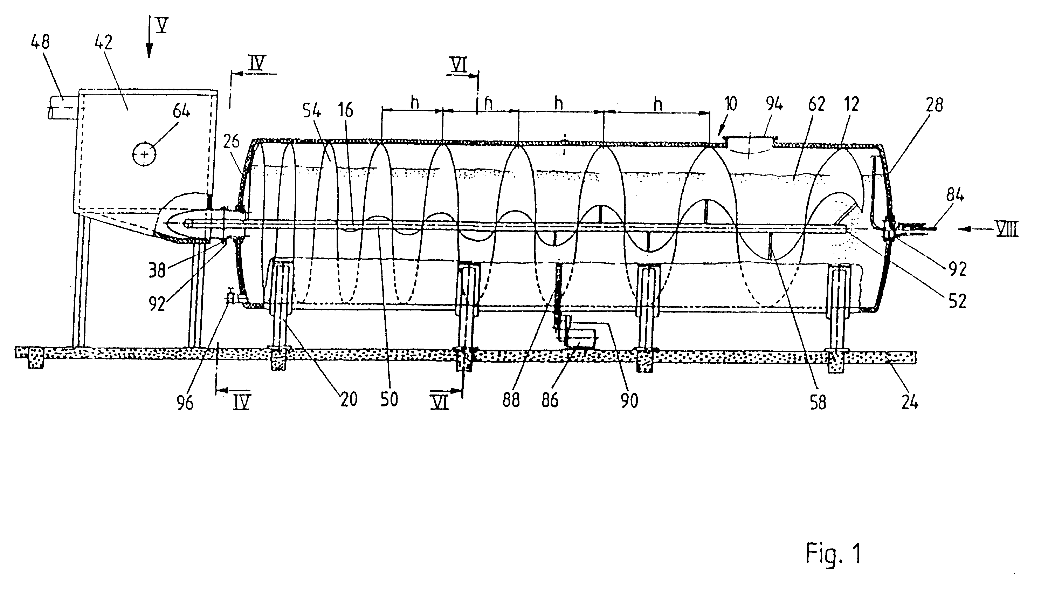

Im Ausführungsbeispiel der Figuren 1 bis 8 ist eine Reaktortrommel 10 mit ihrem zylindrischen Hauptteil 12 so auf mehreren Radial-Wälzlagern 14 gelagert, daß ihre Achse 16 etwa waagrecht verläuft. Die Wälzlager 14, auf deren Rollen 18 am zylindrischen Hauptteil 12 angebrachte Laufringe 20 abgestützt sind, sind in Lagerböcken 22 angeordnet, welche an einem Fundament 24 verankert sind.

Die Reaktortrommel 10 ist an einer Stirnseite durch eine Beschickungswand 26 und an der gegenüberliegenden Stirnseite durch eine Gasentnahmewand 28 abgeschlossen. Sowohl der zylindrische Hauptteil 12 als auch die Beschickungswand 26 und die Gasentnahme 28 sind in Sandwich-Bauweise ausgeführt, wobei eine mittlere Isolierschicht 30 zwischen einer glatten Innenwand 32 aus korrosionsfestem Kunststoff und einer steifen Außenwand 34 eingeschlossen ist.

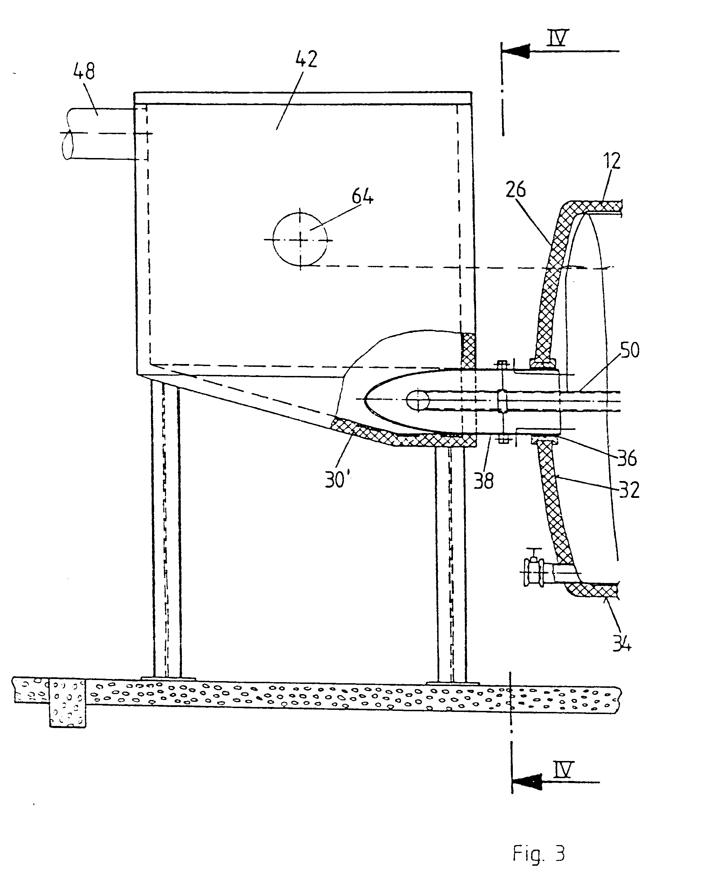

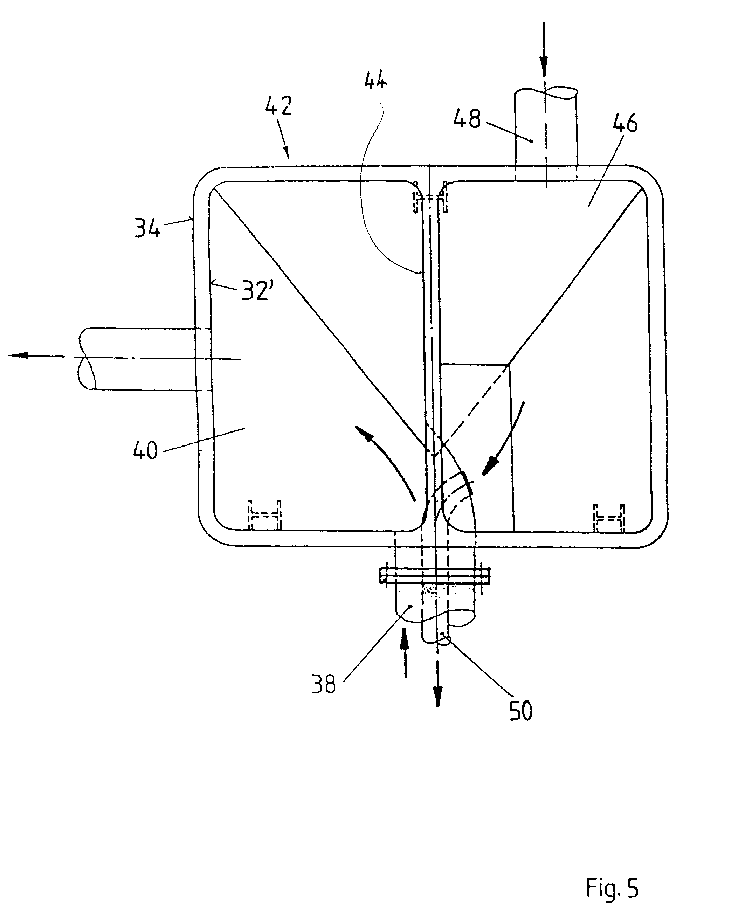

Wie vor allem Figur 3 zeigt, ist in die Beschickungswand 26 der Reaktortrommel 10 eine zentrische Öffnung eingearbeitet, in welcher über ein Radial-Gleitlager 36 ein fester Abgaberohrstutzen 38 abgedichtet gelagert ist. Das aus der Reaktortrommel 10 herausragende Ende des Abgaberohrstutzens 38 mündet, wie vor allem Figur 5 zeigt, in eine Auslaufkammer 40 eines Vorsatzbehälters 42.

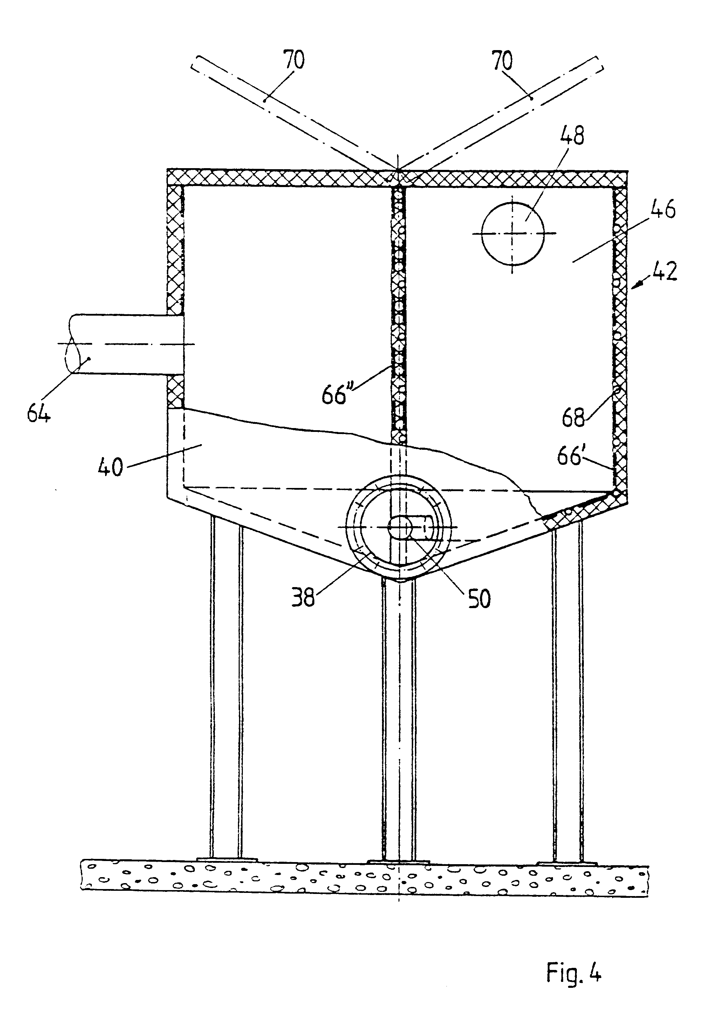

Wie die Figuren 4 und 5 zeigen, ist die Auslaufkammer 40 des Vorsatzbehälters 42 durch eine Trennwand 44 von einer Vorgärkammer 46 abgeteilt. Aus dieser Vorgärkammer 46, in die ein Beschickungsrohr 48 mündet, führt ein Zuführrohr 50 koaxial durch den Abgaberohrstutzen 38 in die Reaktortrommel 10. Das Zuführrohr 50 durchläuft die Reaktortrommel 10 koaxial und endet kurz vor der Gasentnahmewand 28, wo sein Auslauf 52 in die Reaktortrommel 10 mündet.

Um das Zuführrohr 50 herum ist eine Förderschnecke 54 angeordnet, die sowohl mit dem Zuführrohr 50 als auch mit der zylindrischen Innenwand 32 der drehbaren Reaktortrommel 10 verbunden ist. Wie Figur 1 zeigt, nimmt die Steigung h der Förderschnecke zur Gasentnahmewand 28 hin zu. Damit wird erreicht, daß in Richtung zur Gasentnahmewand 28 die Angriffsfläche der Förderschnecke 54 immer größer wird.

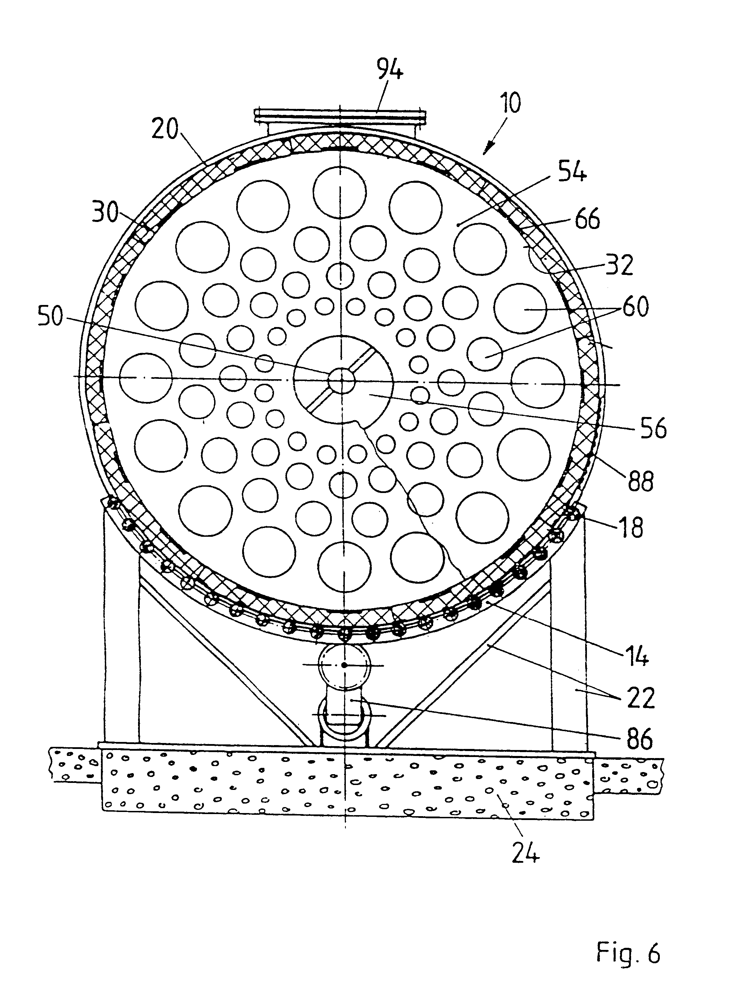

Außerdem weist die Förderschnecke 54 auf dem größten Teil ihrer Länge - mit Ausnahme der ersten Windungen hinter der Beschickungswand 26 - im Kernbereich einen Abstand zum Zuführrohr 50 auf, so daß in Axialrichtung gesehen eine etwa kreisförmige Durchtrittsöffnung 56 (vergl. Figur 6) zwischen der Förderschnecke 54 und dem Zuführrohr 50 frei bleibt. Wie weiterhin Figur 1 zeigt, ist die Durchtrittsöffnung 56 im Längsschnitt durch eine gedachte Kegelfläche begrenzt, deren Kegelspitze in der Nähe der Beschickungswand 26 liegt. Das bedeutet, daß die Durchtrittsöffnung 56 in Richtung zur Gasentnahmewand 28 stetig größer wird.

Im Bereich der Durchtrittsöffnung 56 ist die Förderschnecke 54 durch Stabilisationsstege 58 am Zuführrohr 50 befestigt.

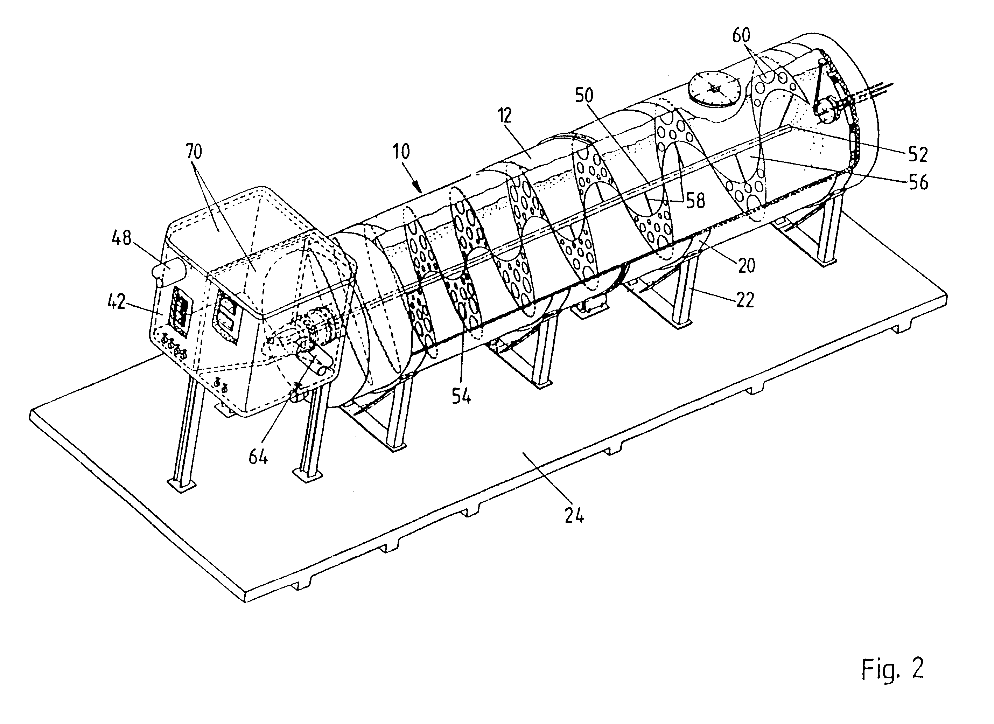

Schließlich zeigen die Figuren 2, 6 und 7, daß in die Förderschnecke 54 kreisförmige Durchbrüche 60 für den axialen Durchtritt von Substrat und Biogas eingearbeitet sind. Die radial äußersten Durchbrüche 60 sind dabei so angeordnet, daß sie in der obersten Stellung mindestens teilweise über der Befüllungshöhe der Reaktortrommel 10 mit dem Substrat 62 liegen, in der untersten Stellung jedoch mit ihrem radial äußersten Rand einen Abstand zur Innenwand 32 der Reaktortrommel 10 halten, so daß Fremdkörper wie Steine oder dergleichen von der Förderschnecke 54 mitgenommen werden und nicht durch die Durchbrüche 60 hindurchtreten können.

In der erwähnten Befüllungshöhe der Reaktortrommel 10 führt aus der Auslaufkammer 40 des Vorsatzbehälters 42 ein Auslaufrohr 64 heraus,durch das das ausgefaulte Substrat abgezogen werden kann. Die Höhe des Auslaufrohres 64 bestimmt aufgrund des Prinzips der kommunizierenden Röhren die Befüllungshöhe der Reaktortrommel 10.

Aus demselben Grund ist das in die Vorgärkammer 46 mündende Beschickungsrohr 48 über der Scheitelhöhe der Reaktortrommel 10 angeordnet, so daß die Befüllung der Reaktortrommel 10 mit frischem Substrat selbsttätig aufgrund des statischen Druckes erfolgt.

Ebenso wie die Reaktortrommel 10 ist auch die Wand des Vorsatzbehälters 42 in Sandwich-Bauweise ausgeführt, wobei zwischen einer glatten Innenwand 32' und einer steifen Außenwand 34' eine Isolierschicht 30' eingeschlossen ist.

In die Wand der Vorgärkammer 46 sind Wärmetauschelemente zur Vorwärmung des frischen Substrates eingebaut, und zwar Flächenwärmetauscher 66' zur Beheizung mit Warmwasser und röhrenförmige Wärmetauscher 68 zur Beheizung mit Warmluft oder Abgas-Restwärme des Systems.

Wie Figur 4 ferner zeigt, sind auch in die Wand der Auslaufkammer 40 Flächenwärmetauscher 66'' eingebaut, die zur Aufnahme der Restwärme des ausgefaulten Substrates dienen und die beispielsweise mit den Flächenwärmetauschern 66' der Vorgärkammer 46 verbunden sein können.

Die Befüllung und die Entleerung des Vorsatzbehälters 42 kann, wie Figur 4 zeigt, auch über von Hand aufklappbare Deckel 70 erfolgen.

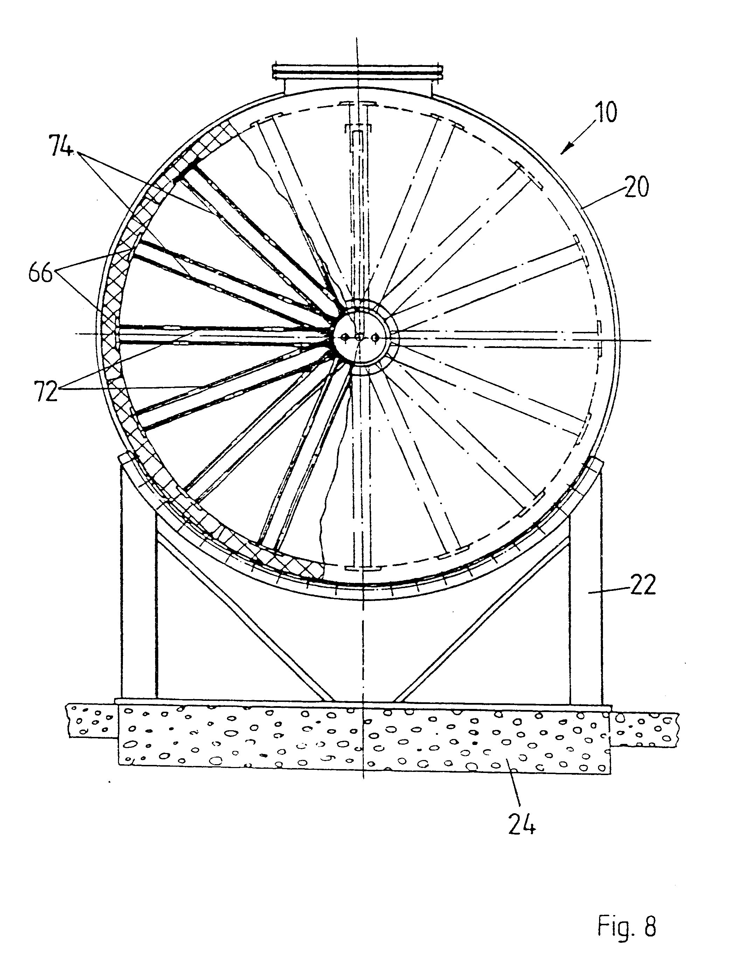

Ähnlich wie beim Vorsatzbehälter 42 besteht auch die in die Trommelwand integrierte Heizung aus Flächenwärmetauschern 66, die sich in Achsrichtung der Reaktortrommel 10 erstrecken und, wie die Figuren 6 und 8 zeigen, in Umfangsrichtung gleichmäßig voneinander beabstandet sind. Jeder Flächenwärmetauscher für das Wärmeübertragungsmedium, im allgemeinen Wasser, enthält ein Vorlaufrohr 72 und ein Rücklaufrohr 74, die den zylindrischen Hauptteil 12 der Reaktortrommel 10 in Achsrichtung durchlaufen und im Bereich der Beschickungswand 26 durch ein U-förmiges Rohrstück miteinander verbunden sind.

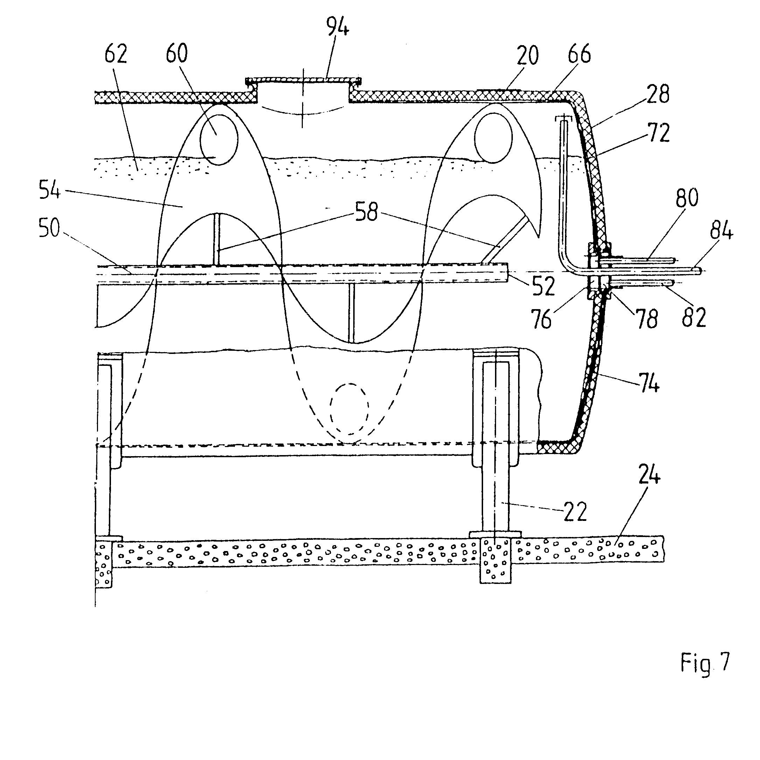

Wie Figur 7 zeigt, ist in der Gasentnahmewand 28 eine stillstehende, zentrische Doppelkammer mit einer Vorlaufkammer 76 und einer Rücklaufkammer 78 angeordnet, die gegen die drehbare Gasentnahmewand 28 gleitend abgedichtet ist. Die Vorlaufkammer 76 ist mit den Vorlaufrohren 72 und die Rücklaufkammer 78 mit den Rücklaufrohren 74 verbunden; in die Vorlaufkammer 76 mündet eine Leitung 80 für den Heizungsvorlauf, während aus der Rücklaufkammer 78 eine Leitung 82 für den Heizungsrücklauf herausführt.

Zentrisch durch die stillstehende Doppelkammer hindurch ist ein Entnahmerohr 84 zum Abziehen des erzeugten Biogases hindurchgeführt, dessen freies Ende in der Reaktortrommel 10 über der Befüllungshöhe liegt. Das durch die Gärung des Substrates 62, in das eine Zellkultur, im allgemeinen Bakterien, eingeführt worden ist, erzeugte Biogas wird über das Entnahmerohr 84 in einen Speicherbehälter abgezogen.

Für den Drehantrieb des Reaktorbehälters 10 ist ein Elektromotor 86 mit einem Untersetzungsgetriebe auf dem Fundament 24 befestigt, der die Reaktortrommel 10 über einen Zahnriemen 88 antreibt. Mit dem Elektromotor 86, der in beiden Drehrichtungen betrieben werden kann, ist eine Regeleinrichtung 90 verbunden, die ihrerseits mit Meßsonden 92 verbunden ist, welche zum Erfassen der Prozeßdaten in die Reaktortrommel 10 eingesetzt sind.

Figur 1 zeigt schließlich, daß die Reaktortrommel 10 mit einem Einstiegloch 94 und mit einem Ablaßhahn 96 ausgerüstet ist.

Wie bereits angedeutet, wird zum Befüllen der Reaktortrommel 10 mit frischem Substrat die Trommel durch den Elektro motor 86 in Gangrichtung der Förderschnecke 54 gedreht, so daß das Substrat aus der Vorgärkammer 46 über das Zuführrohr 50 in den Teil der Reaktortrommel 10 gelangt, der durch die Gasentnahmewand 28 begrenzt ist, wo es von der Förderschnecke 54 erfaßt und in Richtung auf die Beschickungswand 26 gedrückt wird. Dabei erfolgt eine ständige, gute Durchmischung des Substrates 62 zwischen Sinkschicht und Schwimmdecke; gleichzeitig wird das Substrat durch die Flächenwärmetauscher 66 auf die Prozeßtemperatur erwärmt, so daß sich im oberen Teil der Reaktortrommel 10 Biogas bildet. Während des Gärprozesses wird die Reaktortrommel 10 immer wieder hin- und hergedreht, so daß eine gute Durchmischung gewährleistet ist.

Sobald die Meßsonden 92 im Bereich der Beschickungswand 26 feststellen, daß das Substrat in diesem Bereich vollständig ausgefault ist, wird durch eine entsprechende Drehung der Reaktortrommel 10 der vordere Teil des Substrates über den Abgaberohrstutzen 38 in die Auslaufkammer 40 gedrückt. Aufgrund der Durchlauftechnik mit Hilfe der Förderschnecke 54 stimmt die eingefüllte Menge frischen Substrates immer mit der abgegebenen Menge ausgefaulten Substrates überein.

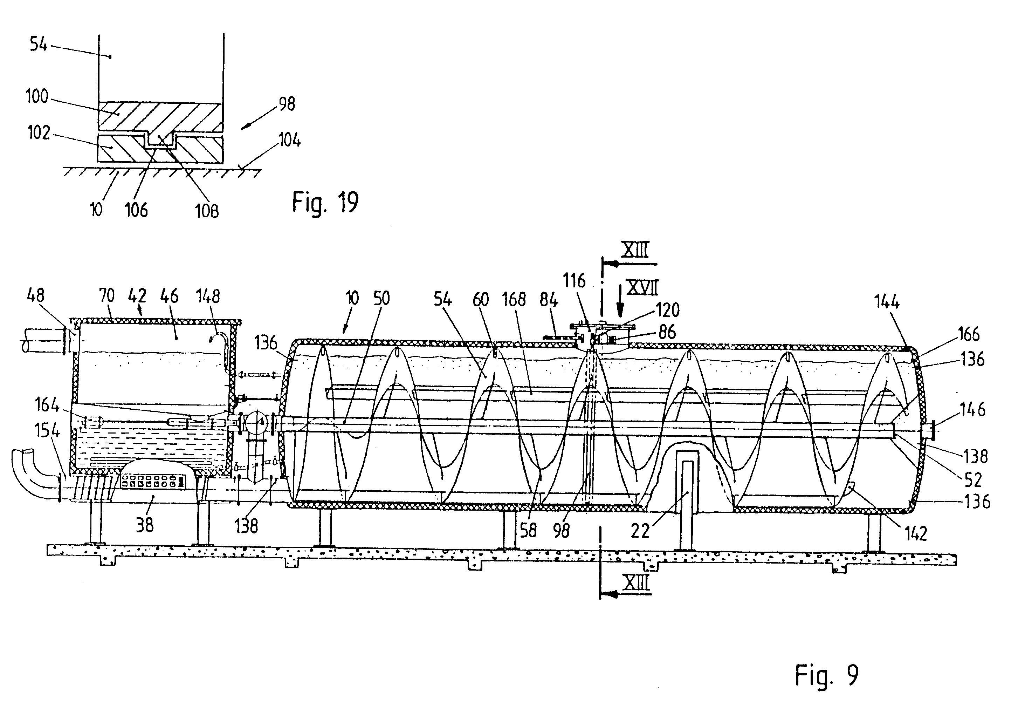

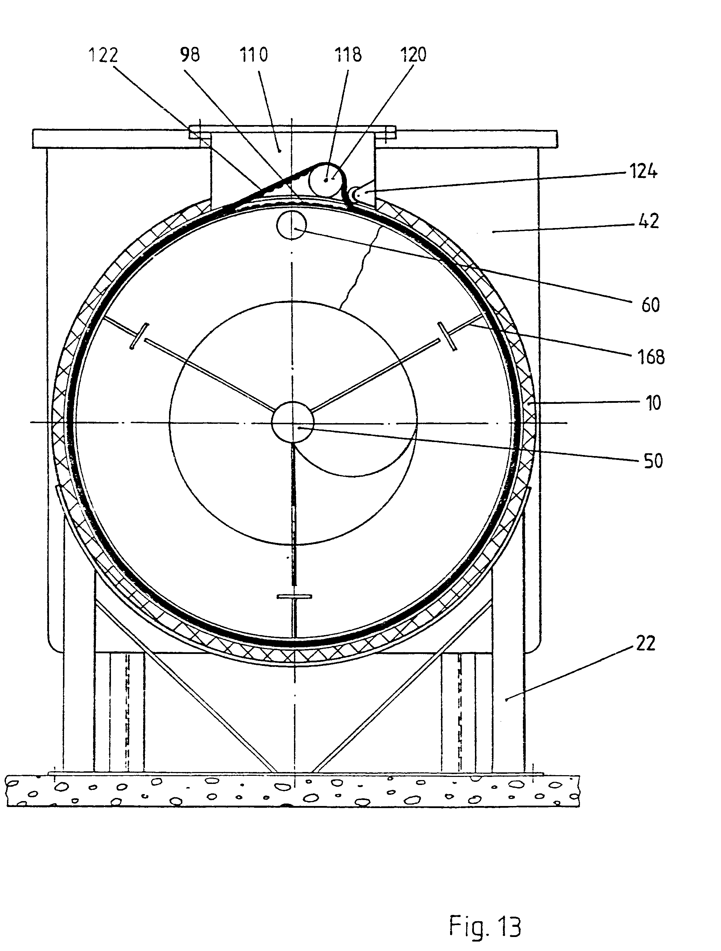

Aufgrund der freien und nicht in einem Behälter oder Becken untergebrachten Anordnung der Reaktortrommel 10 kann diese mit ihrer Länge den geforderten Betriebsbedingungen angepaßt werden, ohne daß ein etwa aus Beton gefertigter Wasserbehälter an die Länge angepaßt werden müßte. Bei der in den Figuren 9 bis 19 gezeigten Ausführungsform ist die Reaktortrommel 10 feststehend, während die Förderschnecke 54 in dieser drehbar gelagert ist. Die Förderschnecke 54 ist mit dem Zuführrohr 50 fest verbunden und in der Reaktortrommel 10 durch Gleitlager 98 gelagert. In Figur 9 ist im mittleren Bereich nur ein Gleitlager 98 gezeigt, es können jedoch je nach Trommelgröße und auftretenden Betriebskräften mehr Gleitlager über die Länge der Förderschnecke 54 verteilt sein.

Wie Figur 19 zeigt, besteht jedes Gleitlager 98 aus einem Innenring 100, der am Außenumfang der Förderschnecke 54 befestigt ist, und aus einem Außenring 102, der an der zylindrischen Innenwand 104 der Reaktortrommel 10 drehbar abgestützt ist. Aus Gründen der Verschleißfestigkeit und der Gleitreibungseigenschaften ist es günstig, wenn der Innenring 100 aus Polyester und der Außenring 102 aus Polyäthylen besteht.

In den Außenring 102 ist eine umlaufende Nut 106 eingearbeitet, in die ein Ringvorsprung 108 des Innenrings 100 eingreift, so daß der schwimmend gelagerte Außenring 102 in Axialrichtung geführt ist.

Die Reaktortrommel 10 weist in ihrem in Längserstreckung mittleren Bereich an der Oberseite eine Einstiegsluke 110 auf. Diese Einstiegsluke 110 ist in eine durch eine Wand 112 gegen die Reaktortrommel 10 abgedichtete Motorkammer 114 und in einen mit dem Trommelinneren verbundenen Gasraum 116 unterteilt.

In der Motorkammer 114 ist der Drehantrieb 86 für die Förderschnecke 54 untergebracht, dessen Antriebswelle 118 mit einem daran befestigten Antriebsritzel 120 in den Gasraum 116 ragt. Das Antriebsritzel 120 kämmt mit einem Zahnriemen 122, der formschlüssig mit dem Schneckenumfang verbunden ist, beispielsweise über eine feste Verzahnung am Außenumfang der Förderschnecke 54. Der Zahnriemen 122 wird durch eine Spannrolle 124 unter Spannung gehalten.

Aus dem Gasraum 116 führt das Entnahmerohr 84 zum Abziehen des erzeugten Biogases heraus. Das durch die Gärung des Substrates erzeugte Biogas wird über das Entnahmerohr 84 in einen nicht gezeigten Speicherbehälter abgezogen.

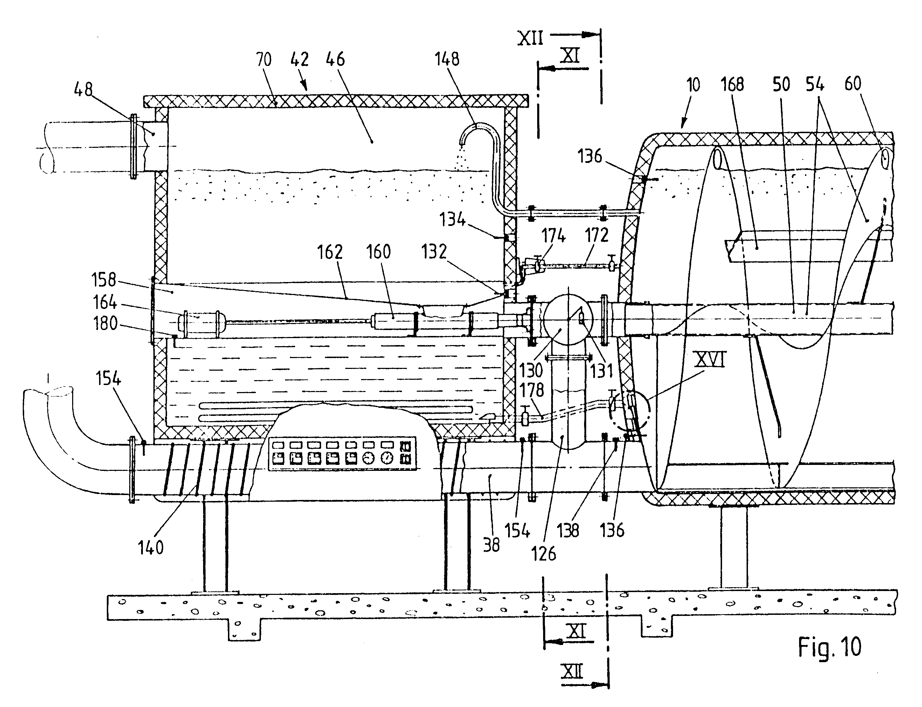

Wie Figur 10 zeigt, ist aus der Beschickungswand 26 der Reaktortrommel 10 das Abgaberohr 38 für das ausgefaulte Substrat herausgeführt. Dieses Abgaberohr 38 ist über eine Kurzschlußleitung 126 mit dem einlaßseitigen, festen Teil 50' des Einlaßrohrstückes verbunden, auf das der in der Reaktortrommel 10 drehbare Teil des Zuführrohres 50 mittels einer Gleitmuffe 128 aufgesteckt ist. An der Mündung des Zuführrohres 50 in die Kurzschlußleitung 126 ist ein Dreiweghahn 130 vorgesehen. Dieser kann durch eine Regeleinrichtung 131 gesteuert werden, welcher Meßsonden 132 und 134 zugeordnet sind, die die Prozeßdaten im Vorsatzbehälter 42 erfassen. Dabei kann die Meßsonde 132 als Temperaturfühler und die Meßsonde 134 zum Messen des pH-Wertes ausgebildet sein.

Mit Hilfe weiterer Meßsonden 136 und 138 kann der als Elektromotor ausgebildete Drehantrieb 86 der Förderschnecke 54 in beiden Drehrichtungen gesteuert werden. Dabei dienen die Meßsonden 136 im Bereich der Beschickungswand 26 und der gegenüberliegenden Stirnwand 28 zur Temperaturmessung, wobei diese Meßwerte über einen Prozeßrechner zur Temperaturüberwachung und Heizungsregelung eingesetzt werden. Die Meßsonden 138 sind chemische Reaktionssonden, wobei die Meßsonde 138 an der zweiten Stirnwand 28 den pH-Wert des frischen Substrates am Auslauf 52 des Zuführrohres 50 auf den eingeleiteten Gärzustand hin analysiert und als Eckwert der Durchlaufphase in der Reaktortrommel 10 zwischen Substrateinlauf und Faulgutaustritt dient. Die Meßsonde 138 im Abgaberohr 38 mißt beispielsweise über den pH-Wert den Ist-Gärzustand, dessen Sollwert im angeschlossenen Prozeßrechner vorgegeben werden kann. In diesem Meßbereich wird je nach dem Vergärungszustand die zeitliche Intervallschaltung der Mischdrehbewegung außer Kraft gesetzt und die Entleerungsdrehbewegung der Förderschnecke 54 so lange aktiviert, bis das vergärte Faulmaterial im vorgegebenen Gärzustand entleert ist. Anschliessend tritt automatisch wieder die Mischregelung in Funktion, in der durch hin-und hergehende Rotation der Förderschnecke 54 das Substrat durchmischt wird.

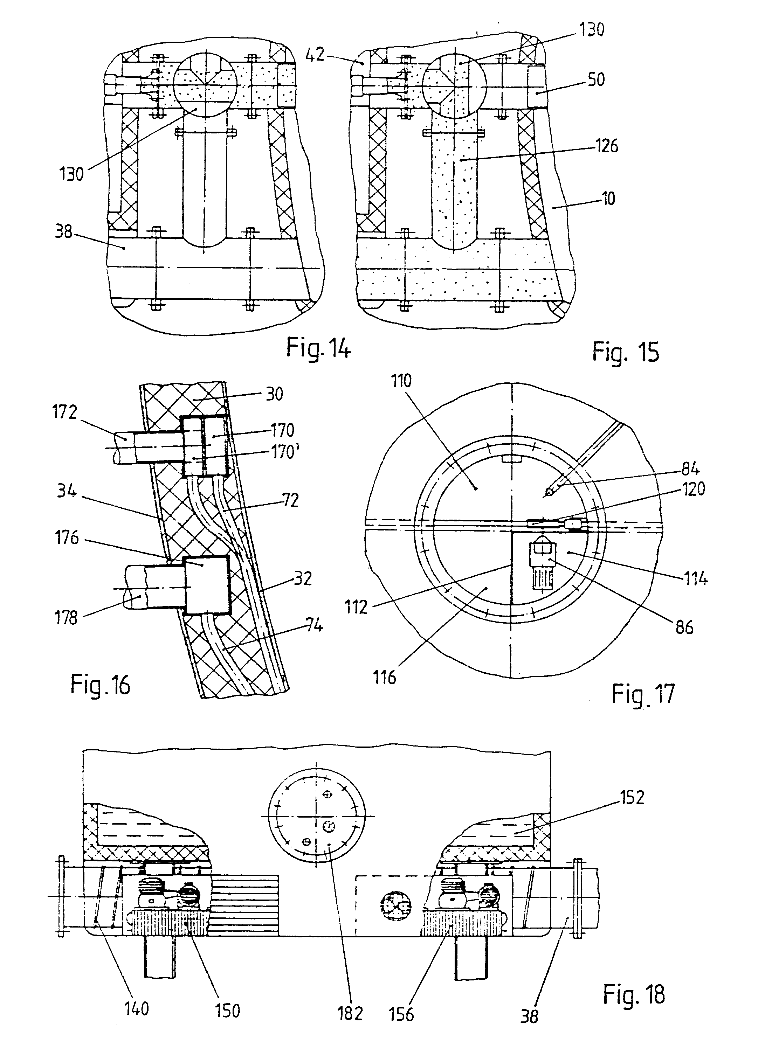

Wie die Figuren 10 und 18 zeigen, ist das dicht unter dem Boden des Vorsatzbehälters 42 verlaufende Abgaberohr 38 mit Wärmetauschelementen 140 zur Aufnahme der Restwärme des ausgefaulten Substrates ausgerüstet. Mittels einer Wärmepumpe 150 kann diese zurückgewonnene Wärme unmittelbar an einen darüberliegenden Warmwasserspeicher 152 abgegeben werden.

Je eine Temperatursonde 154 am Eingang und am Ausgang des wärmerückgewinnenden Teils des Abgaberohres 38 messen den Temperaturabfall und regeln über den bereits erwähnten Prozeßrechner den gewünschten Wärmeentzugsgrad. Eine großzügige Dimensionierung dieses Teils des Abgaberohres 38 ermöglicht einen langsamen Durchfluß und durch eine kontinuierliche Entleerung in kleinen Mengen eine optimale Wärmerückgewinnung. Die Temperaturdifferenz zwischen dem auslaufenden Substrat und dem wärmeren Frischsubstrat - in der Praxis mindestens 10° C - ermöglicht wenigstens den Ausgleich von Strahlungsverlusten.

Eine weitere Wärmepumpe 156, die beispielsweise der Umgebungsluft Wärme entzieht, kann zum Ausgleich des defizitären Wärmebedarfs des Prozesses eingesetzt werden.

Wie vor allem Figur 10 zeigt, ist der Vorsatzbehälter 42 durch ein horizontal verlaufendes Trennelement in die obere Vorgärkammer 46 und den unteren Warmwasserspeicher 152 unterteilt. Das Trennelement ist dabei als ein von außen zugänglicher Aufnahmeschacht 158 für eine Substratpumpe 160 ausgebildet. Der Ausgang dieser Substratpumpe 160 ist mit dem festen Teil 50' des Zuführrohres verbunden, während in deren Eingang ein die Vorgärkammer 46 nach unten begrenzender, nicht isolierter Trichterboden 162 mündet. Durch die aufsteigende Wärme des Warmwasserspeichers 152 wird das Frischsubstrat in der Vorgärkammer 46 bereits vorgewärmt.

Zum Antrieb der Substratpumpe 160 ist im Aufnahmeschacht 158 ein Elektromotor 164 angeordnet.

Im Bereich der zweiten Stirnwand 28 ist in dem Trommelreaktor 10 ein Sicherheitsschalter 166 angeordnet, der die Substratpumpe 160 bei Erreichen eines maximalen Füllpegels ausschaltet.

Aus den Figuren 9, 10 und 13 geht hervor, daß an den Schneckengängen der Förderschnecke 54 im radial äußeren Bereich in Axialrichtung verlaufende, profilierte Durchmischungsstangen 168 befestigt sind. Diese T-förmigen, axial miteinander verbundenen Durchmischungsstangen 168 verhindern die Bildung einer Schwimmdecke und befördern mögliche Sinkschichten während der Rotation der Förderschnecke 54 nach oben.

In Figur 9 ist angedeutet, daß an der Förderschnecke 54 Mischelemente 142 befestigt sein können, die so ausgebildet und angeordnet sind, daß sie das Substrat in der Reaktortrommel 10 sowohl bei einer Rechtsdrehung als auch bei einer Linksdrehung der Förderschnecke 54 gut durchmischen.

Die zweite Stirnwand 28 ist, wie in Figur 1 angedeutet, durch eine Flanschverbindung 144 abnehmbar an der Reaktortrommel 10 befestigt. Für den Bedarfsfall weist diese zweite Stirnwand 28 eine Ersatz-Zuführeinrichtung 146 für frisches Substrat auf.

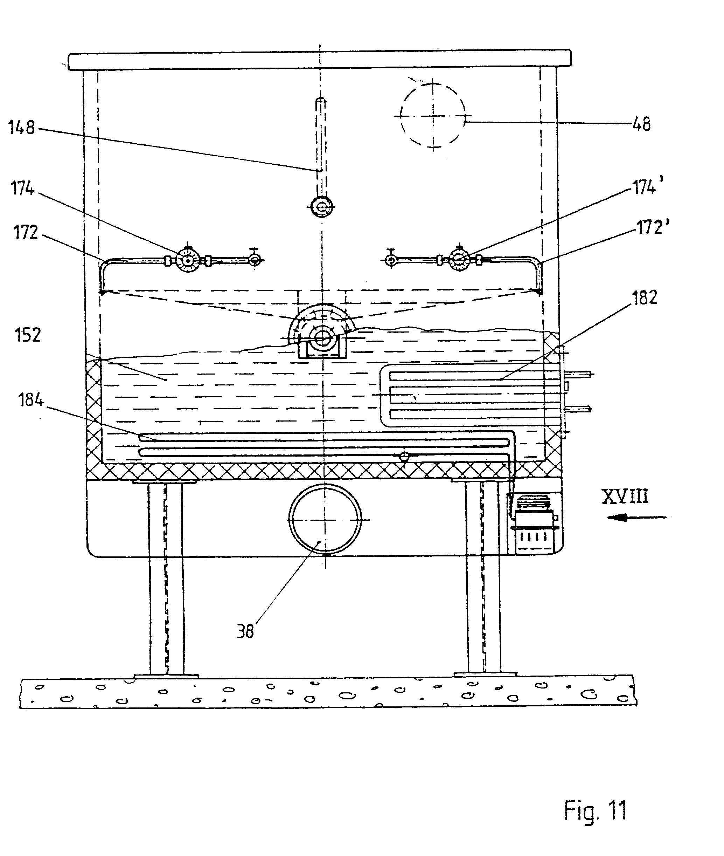

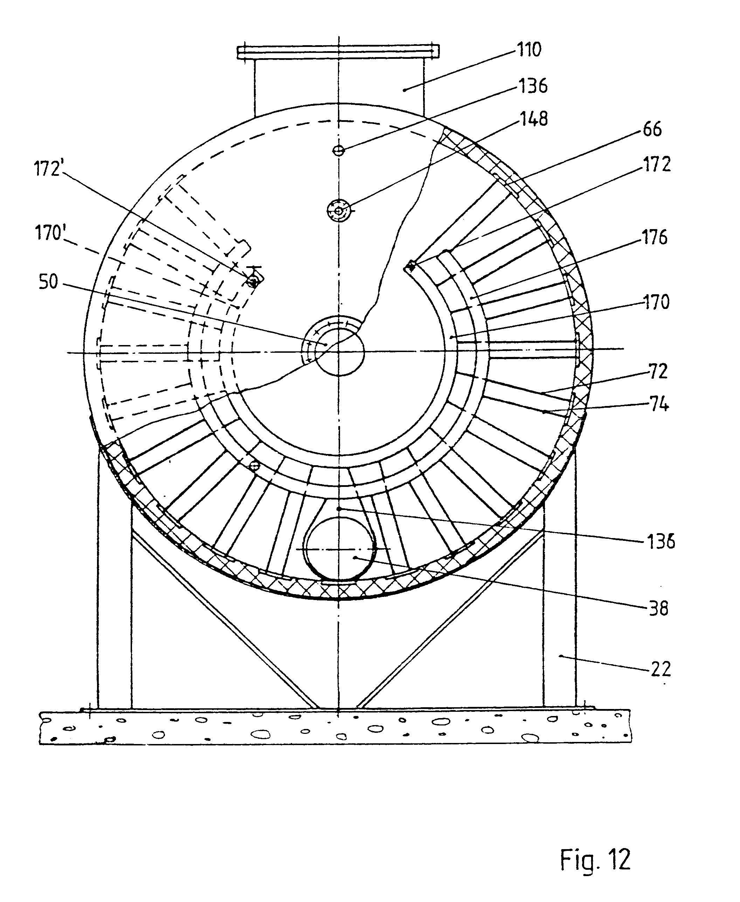

In den Figuren 12 und 16 ist zu erkennen, daß jeder Flächenwärmetauscher 66, der in die Trommelwand integriert ist, ein Vorlaufrohr 72 und ein Rücklaufrohr 74 enthält. In der Beschickungswand 26 münden die Vorlaufrohre 72 abwechselnd in je eine von zwei axial hintereinander angeordneten, ringförmigen Vorlaufkammern 170 bzw. 170', wobei, wie Figur 12 zeigt, die Vorlaufkammer 170 oben rechts über eine Verbindungsleitung 172 mit einer Umwälzpumpe 174 in der in Figur 11 oberen, linken Ecke mit dem Warmwasserspeicher 152 verbunden ist; die andere Vorlaufkammer 170' führt über die in Figur 12 linke Verbindungsleitung 172' in die in Figur 11 rechte obere Ecke des Warmwasserspeichers 152.

Bei dieser Anordnung werden die beiden ringförmigen Vorlaufkammern 170 und 170' im Gegenstrom mit Warmwasser versorgt, wobei jede Verbindungsleitung 172 und 172' abwechselnd jeden zweiten Flächenwärmetauscher 66 versorgt. Nach dem Durchlauf durch die Flächenwärmetauscher 66 gelangt das Warmwasser über die Rücklaufrohre 74 in eine ringförmige Rücklaufkammer 176 in der Beschickungswand 26 und von dieser über eine Rückleitung 178 zurück in den Warmwasserspeicher 152.

Zur Regelung der Wärmepumpen 150 und 156 ist der Warmwasserspeicher 152 mit einer Temperaturmeßsonde 180 ausgerüstet.

Figur 11 zeigt, daß in den Warmwasserspeicher 152 ein Wärmetauschelement 182 eingesetzt sein kann, das beispielsweise durch eine externe Warmwasserzufuhr, warmes Abgas, Abluft oder Elektroheizstäbe gespeist werden kann.

Die Wärmepumpen 150 und 156 beheizen das Wasser des Warmwasserspeichers 152 über Heizschlangen 184.

Zum Befüllen der Reaktortrommel 10 mit frischem Substrat wird die Substratpumpe 160 eingeschaltet, welche das Substrat aus der Vorgärkammer 46 in das Zuführrohr 50 drückt; dabei dreht sich die Förderschnecke 54, so daß sie das Einziehen des Substrates begünstigt.

Während dieses Vorgangs befindet sich der Dreiweghahn 130 normalerweise in der in Figur 14 gezeigten Stellung, in der die Mündung in die Kurzschlußleitung 126 verschlossen ist. Falls jedoch die Meßsonden 132 und 134 feststellen, daß das eingezogene Substrat bereits verdorben ist, wird über die Regeleinrichtung 131 der Dreiweghahn 130 in die Stellung der Figur 15 gedreht, so daß das Zuführrohr 50 gesperrt ist und das Substrat über die Kurzschlußleitung 126 in das Abgaberohr 38 zurückgeleitet wird.

Beim Ausschieben des vergorenen Substrates drückt die Förderschnecke 54 einen kleinen Teil davon durch die dünne Rücklaufleitung 148 in die Vorgärkammer 46, so daß das darin befindliche, frische Substrat mit diesem vergorenen Substrat geimpft wird. Das hat den Vorteil, daß das frische Substrat früher vergärt, wodurch die Verweildauer in der Reaktortrommel 10 verkürzt wird.

Die in den Figuren 9 bis 19 gezeigte Vorrichtung ist dazu geeignet, mit weiteren Vorrichtungen derselben Bauart hintereinandergeschaltet zu werden, um unterschiedliche Gärprozesse ablaufen zu lassen. Hierzu kann der Ersatz-Zuführanschluß 146, der dann als Auslauf dient, mit dem Zuführrohr 50 einer folgenden Reaktortrommel 10 verbunden werden.

## Patentansprüche

1. Vorrichtung zur Erzeugung von Biogas aus organischem Substrat mit einer dieses aufnehmenden, eine waagrechte Achse aufweisenden Reaktortrommel mit Rührwerk, die an einer ersten Stirnseite (Beschickungswand) eine Zuführeinrichtung für das frische Substrat und eine Abführeinrichtung für das ausgefaulte Substrat aufweist und mit einer Entnahmeeinrichtung für das erzeugte Biogas sowie mit einer Heizung zur Zuführung der Prozeßwärme ausgerüstet ist, **dadurch gekennzeichnet,** daß für die Zuführung des frischen Substrates (62) ein mit einem Einfüllbehälter an der Beschickungswand (26) verbundenes Zuführrohr (50) die Reaktortrommel (10) koaxial bis nahe an die gegenüberliegende, zweite Stirnwand (28) durchquert, wo sein Auslauf (52) in die Reaktortrommel (10) mündet, daß um das Zuführrohr (50) herum eine der Länge der Reaktortrommel (10) entsprechende Förderschnecke (54) angeordnet ist, deren Abgabeseite an der Beschickungswand (26) zu einem Abgaberohr (38) führt, und daß die Wand der Reaktortrommel (10) mit der Heizung zur Zuführung der Prozeßwärme ausgerüstet ist.

2. Vorrichtung nach Anspruch 1, dadurch gekennzeichnet, daß die Förderschnecke (54) wenigstens auf dem größten Teil ihrer Längserstreckung im Kernbereich einen Abstand zum Zuführrohr (50) aufweist derart, daß in Axialrichtung gesehen eine etwa kreisförmige Durchtrittsöffnung (56) zwischen der Förderschnecke (54) und dem Zuführrohr (50) frei bleibt.

3. Vorrichtung nach Anspruch 2, dadurch gekennzeichnet, daß die Durchtrittsöffnung (56) im Längsschnitt durch eine gedachte Kegelfläche begrenzt ist, deren Kegelspitze in der Nähe der Beschickungswand (26) liegt.

4. Vorrichtung nach einem der vorhergehenden Ansprüche, dadurch gekennzeichnet, daß die Steigung (h) der Förderschnecke (54) zur zweiten Stirnwand (28) hin zunimmt

5. Vorrichtung nach einem der vorhergehenden Ansprüche, dadurch gekennzeichnet, daß zur Relativdrehung zwischen Reaktortrommel (10) und Förderschnecke (54) ein in beiden Drehrichtungen steuerbarer Drehantrieb (86) vorgesehen ist.

6. Vorrichtung nach Anspruch 5, dadurch gekennzeichnet, daß der Drehantrieb (86) mit einer Regeleinrichtung (90) verbunden ist, der in die Reaktortrommel (10) eingesetzte Meßsonden (92) zum Erfassen der Prozeßdaten zugeordnet sind.

7. Vorrichtung nach Anspruch 5 oder 6, dadurch gekennzeichnet, daß der Drehantrieb (86) für die Förderschnecke (54) im in Längserstreckung mittleren Bereich der stillstehenden Reaktortrommel (10) befestigt ist.

8. Vorrichtung nach Anspruch 7, dadurch gekennzeichnet, daß der Drehantrieb (86) in einer gegen die Reaktortrommel (10) abgedichteten Motorkammer (114) angeordnet ist, aus der die Antriebswelle (118) mit einem daran befestigten Antriebsrad (120) in einen Gasraum (116) an der Oberseite der Reaktortrommel (10) ragt.

9. Vorrichtung nach Anspruch 8, dadurch gekennzeichnet, daß das Antriebsrad (120) als Ritzel ausgebildet ist und mit einem formschlüssig mit dem Schneckenumfang verbundenen Zahnriemen (122) kämmt.

10. Vorrichtung nach Anspruch 8 oder 9, dadurch gekennzeichnet, daß aus dem Gasraum (116) ein Entnahmerohr (84) zum Abziehen des erzeugten Biogases herausführt.

11. Vorrichtung nach einem der vorhergehenden Ansprüche, dadurch gekennzeichnet, daß aus der Beschickungswand (26) der Reaktortrommel (10) das Abgaberohr (38) für das ausgefaulte Substrat herausführt, das über eine Kurzschlußleitung (126) mit der Einlaßseite des Zuführrohres (50) verbunden ist, wobei an der Mündung des Zuführrohres (50) in die Kurzschlußleitung (126) ein Dreiweghahn (130) vorgesehen ist.

12. Vorrichtung nach Anspruch 11, dadurch gekennzeichnet, daß der Dreiweghahn (130) durch eine Regeleinrichtung steuerbar ist, der Meßsonden (132, 134) zum Erfassen der Prozeßdaten im Zuführbereich des frischen Substrates zugeordnet sind.

13. Vorrichtung nach einem der vorhergehenden Ansprüche, dadurch gekennzeichnet, daß die drehbare Förderschnecke (54) durch Gleitlager (98) an der zylindrischen Innenwand (104) der Reaktortrommel (10) gelagert ist.

14. Vorrichtung nach Anspruch 13, dadurch gekennzeichnet, daß jedes Gleitlager (98) aus einem am Außenumfang der Förderschnecke (54) befestigten Innenring (100) und aus einem an der Innenwand (104) der Reaktortrommel (10) drehbar abgestützten Außenring (102) besteht, er zu seiner axialen Führung mit dem Innenring (100) in Eingriff ist.

15. Vorrichtung nach einem der vorhergehenden Ansprüche, dadurch gekennzeichnet, daß die Förderschnecke (54) Durchbrüche (60) für den axialen Durchtritt von Biogas und Substrat aufweist.

16. Vorrichtung nach Anspruch 15, dadurch gekennzeichnet, daß auf jedem Schneckengang ein Durchbruch (60) im radial äußeren Bereich vorgesehen ist, daß alle Durchbrüche (60) in axialer Richtung miteinander fluchten und daß der Drehantrieb (86) über einen Steuerschalter abschaltbar ist derart, daß die Durchbrüche (60) bei Abschaltung der Relativdrehung zwischen Reaktortrommel und Förderschnecke (54) in deren Scheitelbereich liegen.

17. Vorrichtung nach einem der vorhergehenden Ansprüche, dadurch gekennzeichnet, daß an den Schneckengängen der Förderschnecke (54) im radial äußeren Bereich in Axialrichtung verlaufende, profilierte Durchmischungsstangen (168) befestigt sind.

18. Vorrichtung nach einem der vorhergehenden Ansprüche, dadurch gekennzeichnet, daß an der Förderschnecke (54) Mischerschaufeln (142) zum Durchmischen des Substrates in beiden Drehrichtungen der Förderschnecke (54) befestigt sind.

19. Vorrichtung nach einem der vorhergehenden Ansprüche, dadurch gekennzeichnet, daß die zweite Stirnwand (28) der Reaktortrommel (10) eine Ersatz-Zuführeinrichtung (146) für das frische Substrat aufweist.

20. Vorrichtung nach einem der vorhergehenden Ansprüche, dadurch gekennzeichnet, daß die zweite Stirnwand (28) abnehmbar an der Reaktortrommel (10) befestigt ist.

21. Vorrichtung nach einem der vorhergehenden Ansprüche, dadurch gekennzeichnet, daß die Reaktortrommel (10) an ihrer Beschickungswand (26) mit einer Rücklaufleitung (148), die etwa in Höhe des Füllpegels aus der Reaktortrommel (10) herausführt, mit dem als Vorgärkammer (46) ausgebildeten Einfüllbehälter verbunden ist, von dem aus das frische Substrat in das Zuführrohr (50) gelangt, wobei der Querschnitt der Rücklaufleitung (148) wesentlich kleiner ist als derjenige des Abgaberohres (38).

22. Vorrichtung nach einem der vorhergehenden Ansprüche, dadurch gekennzeichnet, daß die Wand des Trommelreaktors (10) und/oder des Einfüllbehälters in Sandwich-Bauweise mit einer mittleren Isolierschicht (30) und einer glatten Innenwand (32) aus korrosionsfestem Kunststoff ausgeführt ist.

23. Vorrichtung nach einem der vorhergehenden Ansprüche, dadurch gekennzeichnet, daß der Einfüllbehälter durch ein Trennelement in eine obere Gärkammer (46) und eien unteren Warmwasserspeicher (152) unterteilt ist.

24. Vorrichtung nach Anspruch 23, dadurch gekennzeichnet, daß das Trennelement ein von außen zugänglicher, horizontaler Aufnahmeschacht (158) für eine Substratpumpe (160) ist, deren Ausgang mit dem Zuführrohr (50) verbunden ist und in deren Eingang ein die Vorgärkammer (46) nach unten begrenzender, nicht isolierter Trichterboden (162) mündet.

25. Vorrichtung nach Anspruch 24, dadurch gekennzeichnet, daß in der Reaktortrommel (10) ein die Substratpumpe (160) bei Erreichen eines maximalen Füllpegels ausschaltender Sicherheitsschalter (166) angeordnet ist.

26. Vorrichtung nach einem der vorhergehenden Ansprüche, dadurch gekennzeichnet, daß die in die Trommelwand integrierte Heizung aus Flächenwärmetauschern (66) besteht, die sich in Achsrichtung der Reaktortrommel (10) erstrecken und in Umfangsrichtung gleichmäßig voneinander beabstandet sind.

27. Vorrichtung nach Anspruch 26, dadurch gekennzeichnet, daß jeder Flächenwärmetauscher (66) für das Wärmeübertragungsmedium ein Vorlaufrohr (72) und ein Rücklaufrohr (74) enthält, daß in der Beschickungswand (26) die Vorlaufrohre (72) in wenigstens eine Vorlaufkammer (170) und die Rücklaufrohre (74) in eine Rücklaufkammer (176) münden und daß die Vorlaufkammer (170) und die Rücklaufkammer (176) mit dem Warmwasserspeicher (152) verbunden sind.

28. Vorrichtung nach Anspruch 27, dadurch gekennzeichnet, daß zwei ringförmige, konzentrische Vorlaufkammern (170, 170') vorgesehen sind, in die das Warmwasser im Gegenstrom läuft und von denen jede mit den Vorlaufrohren (72) jedes zweiten Flächenwärmetauschers (66) verbunden ist.

29. Vorrichtung nach einem der vorhergehenden Ansprüche, dadurch gekennzeichnet, daß die Wand des Abgaberohres (38) Wärmetauschelemente (140) zur Aufnahem der Restwärme des ausgefaulten Substrates für die Wärmezufuhr zum Warmwasserspeicher (152) und/oder zur Vorgärkammer (46) aufweist.

30. Vorrichtung nach einem der vorhergehenden Ansprüche, gekennzeichnet durch wenigstens eine Wärmepumpe (150, 156) für die Wärmezufuhr zum Warmwasserspeicher (152) und/oder zur Vorgärkammer (46).

31. Vorrichtung nach einem der Ansprüche 1 bis 6, 15 bis 20 und 22 bis 30, dadurch gekennzeichnet, daß die nicht drehbare Förderschnecke (54) sowohl an der zylindrischen Innenwand (32) der drehbaren Reaktortrommel (10) als auch an dem Zuführrohr (50) befestigt ist.

32. Vorrichtung nach Anspruch 31, dadurch gekennzeichnet, daß das Abgaberohr (38) zentrisch in der Beschickungswand (26) gelagert ist, wobei das Zuführrohr (50) konzentrisch im Abgaberohr (38) verläuft.

33. Vorrichtung nach Anspruch 31 oder 32, dadurch gekennzeichnet, daß die Reaktortrommel (10) mit ihrem zylindrischen Hauptteil (12) auf Radial-Wälzlagern (14) drehbar abgestützt ist.

34. Vorrichtung nach einem der Ansprüche 31 bis 33, dadurch gekennzeichnet, daß in der zweiten Stirnwand (28) eine stillstehende, zentrische Doppelkammer angeordnet ist, die gegen diese drehbare Stirnwand (28) gleitend abgedichtet ist und von der eine Vorlaufkammer (76) mit den Vorlaufrohren (72) und eine Rücklaufkammer (78) mit den Rücklaufrohren (74) verbunden ist.

35. Vorrichtung nach einem der Ansprüche 31 bis 34, dadurch gekennzeichnet, daß das Zuführrohr (52) und das Abgaberohr (38) in einen Vorsatzbehälter (42) münden, der durch eine Trennwand (44) in den mit dem Zuführrohr (50) verbundenen, als Vorgärkammer (46) ausgebildeten Einfüllbehälter und in eine mit dem Abgaberohr (38) verbundenen Auslaufkammer (40) unterteilt ist.

36. Vorrichtung nach Anspruch 35, dadurch gekennzeichnet, daß aus der Auslaufkammer (40) ein Auslaufrohr (64) in einer Höhe herausführt, die der gewünschten Befüllungshöhe der Reaktortrommel (10) entspricht.

37. Vorrichtung nach Anspruch 35 oder 36, dadurch gekennzeichnet, daß die Vorgärkammer (46) mindestens eine Beschickungsöffnung (48) aufweist, die über der Scheitelhöhe der Reaktortrommel (10) liegt.

38. Vorrichtung nach einem der vorhergehenden Ansprüche, dadurch gekennzeichnet, daß in die Wand der Vorgärkammer (46) Wärmetauschelemente (66',68) zur Vorwärmung des frischen Substrates eingebaut sind.

39. Vorrichtung nach einem der Ansprüche 35 bis 38, dadurch gekennzeichnet, daß in die Wand der Auslaufkammer (40) Wärmetauschelemente (66'') zur Aufnahme der Restwärme des ausgefaulten Substrates eingebaut sind.

**Claims**

1. An apparatus for producing biogas from organic substrate material comprising a reactor drum for receiving the substrate, said reactor drum having a horizontal axis and an agitating mechanism and a first end face (feed wall) of said reactor drum being provided with means for feeding fresh substrate and means for discharging the fermented substrate, and further comprising means for withdrawing the produced biogas, and a heating for supplying the process heat, **characterized** in that for feeding fresh substrate material (62) a feed pipe (50) connected to a charging container adjacent the feed wall (26) extends coaxially through said reactor drum (10) into the vicinity of the opposite, second end wall (28) where the outlet (52) of said feed pipe (50) opens into said reactor drum (10), that a screw conveyor (54) corresponding to the length of said reactor drum (10) is disposed about said feed pipe (50), the delivery side of said screw conveyor (54) leads, at said feed wall (26), to a discharge pipe (38), and that the wall of said reactor drum (10) is equipped with a heating for supplying the process heat.

2. An apparatus according to claim 1, **characterized** in that in the central portion a distance is provided between said screw conveyor (54) and said feed pipe (50) at least along a mayor portion of the longitudinal extension of said screw conveyor (54) such that, viewed in the axial direction, an approximately circular aperture (56) is provided between said screw conveyor (54) and said feed pipe (50).

3. An apparatus according to claim 2, **characterized** in that said aperture (56) is, in a longitudinal sectional view, defined by a fictitious conical surface the apex of which is disposed near said feed wall (26).

4. An apparatus according to anyone of the preceding claims, **characterized** in that the pitch (h) of said screw conveyor (54) increases toward said second end wall (28).

5. An apparatus according to anyone of the preceding claims, **characterized** in that a rotational drive (86) which is controllable in both directions of rotation is provided to provide a relative rotation between said reactor drum (10) and said screw conveyor (54).

6. An apparatus according to claim 5, **characterized** in that said rotational drive (86) is coupled to a control means (90) to which measuring probes (92) are associated which are disposed in said reactor drum (10) for picking up the process data.

7. An apparatus according to claim 5 or 6, **characterized** in that said rotational drive (86) for said screw conveyor (54) is mounted in the central region of the longitudinal extension of said stationary reactor drum (10).

8. An apparatus according to claim 7, **characterized** in that said rotational drive (86) is located in a motor compartment (114) sealed against said reactor drum (10), the drive shaft (118) with a drive wheel (120) secured thereto extending from said motor compartment (114) into a gas space (116) on the upper side of said reactor drum (10).

9. An apparatus according to claim 8, **characterized** in that said drive wheel (120) is a pinion and meshes with a toothed belt (122) positively connected to the screw conveyor circumference.

10. An apparatus according to claim 8 or 9, **characterized** in that a withdrawing pipe (84) for withdrawing the produced biogas leads out of said gas space (116).

11. An apparatus according to anyone of the preceding claims, **characterized** in that from said feed wall (26) of said reactor drum (10) the discharge pipe (38) for fermented substrate extends, said discharge pipe (38) being connected via a bypass conduit (126) to the inlet side of said feed pipe (50), a three-way valve (130) being provided at the location where said feed pipe (50) opens into said bypass conduit (126).

12. An apparatus according to claim 11, **characterized** in that said three-way valve (130) is controllable by a control means which has assigned thereto measuring probes (132, 134) for picking up process data in the region of feed of fresh substrate.

13. An apparatus according to anyone of the preceding claims, **characterized** in that said rotatable screw conveyor (54) is supported by slide bearings (98) at the cylindrical inner wall (104) of said reactor drum (10).

14. An apparatus according to claim 13, **characterized** in that each slide bearing (98) comprises an inner ring (100) secured to the outer circumference of said screw conveyor (54) and an outer ring (102) which is rotatably supported by the inner wall (104) of said reactor drum (10) and engages said inner ring (100) for axial guidance.

15. An apparatus according to anyone of the preceding claims, **characterized** in that said screw conveyor (54) has holes (60) for biogas and substrate material to pass therethrough in the axial direction.

16. An apparatus according to claim 15, **characterized** in that each convolution is provided with a hole (60) in the radially outer portion thereof, that all holes (60) are axially aligned with each other, and that said rotational drive (86) can be switched off by means of a control switch such that said holes (60) are adjacent the apex of said screw conveyor (54) when the relative movement between said reactor drum and said screw conveyor (54) stops.

17. An apparatus according to anyone of the preceding claims, **characterized** in that in the radially outer portions of the convolutions of said screw conveyor (54) there are provided axially extending profiled mixing rods (168).

18. An apparatus according to anyone of the preceding claims, **characterized** in that on said screw conveyor (54) there are mounted mixing blades (142) for mixing the substrate in both directions of rotation of said screw conveyor (54).

19. An apparatus according to anyone of the preceding claims, **characterized** in that said second end wall (28) of said reactor drum (10) is provided with an additional feed means (146) for feeding fresh substrate.

20. An apparatus according to anyone of the preceding claims, **characterized** in that said second end wall (28) is detachably secured to said reactor drum (10).

21. An apparatus according to anyone of the preceding claims, **characterized** in that the reactor drum (10) is provided at its feed wall (26) with a return conduit (148) which leads out of said reactor drum (10) at approximately the height of the filling level and which connects said reactor drum (10) to the charging container which is designed as a pre-fermenting chamber (46) and from which the substrate gets into said feed pipe (50), the cross-sectional dimension of said return conduit (148) being substantially smaller than that of said discharge pipe (38).

22. An apparatus according to anyone of the preceding claims, **characterized** in that the wall of said reactor drum (10) and/or said charging container are of the sandwich construction type having a intermediate insulating layer (30) and a smooth inner wall (32) made of a corrosion-resistant plastic material.

23. An apparatus according to anyone of the preceding claims, **characterized** in that said charging container is divided by a partitioning element into an upper fermenting chamber (46) and a lower hot-water tank (152).

24. An apparatus according to claim 23, **characterized** in that said partitioning element is an externally accessible horizontal compartment (158) accommodating a substrate pump (160) having an outlet connected to said feed pipe (50) and an inlet into which opens a non-insulated funnel-shaped bottom (162) which defines the lower boundary of said pre-fermenting chamber (46).

25. An apparatus according to claim 24, **characterized** in that in said reactor drum (10) there is provided a safety switch (166) which switches said substrate pump (160) off when a maximum filling level has been reached.

26. An apparatus according to anyone of the preceding claims, **characterized** in that said heating built in said drum wall consists of laminar heat exchangers (66) which extend in the axial direction of said reactor drum (10) and which are uniformly spaced from each other in the circumferential direction.

27. An apparatus according to claim 26, **characterized** in that each laminar heat exchanger (66) for the heat transfer medium comprises a forward flow pipe (72) and a return flow pipe (74), that said forward flow pipes (72) open in said feed wall (26) into at least one forward flow chamber (170) and said return flow pipes (74) into a return flow chamber (176), and that said forward flow chamber (170) and said return flow chamber (176) are connected to said hot-water tank (152).

28. An apparatus according to claim 27, **characterized** in that there are provided two annular concentric forward flow chambers (170, 170') into which water flows in countercurrent flow fashion and each of which is connected to said forward flow pipes (72) of each other laminar heat exchanger (66).

29. An apparatus according to anyone of the preceding claims, **characterized** in that the wall of said discharge pipe (38) is provided with heat exchange elements (140) for absorbing the residual heat of the fermented substrate for the supply of heat to said hot-water tank (152) and/or said pre-fermenting chamber (46).

30. An apparatus according to anyone of the preceding claims, **characterized** by at least one heat pump (150, 156) for supplying heat to said hot-water tank (152) and/or said pre-fermenting chamber (46).

31. An apparatus according to anyone of claims 1 to 6, 15 to 20 and 22 to 30, **characterized** in that the non-rotatable screw conveyor (54) is secured to the cylindrical inner wall (32) of the rotatable reactor drum (10) as well as to said feed pipe (50).

32. An apparatus according to claim 31, **characterized** in that said discharge pipe (38) is centrally located in said feed wall (26), with said feed pipe (50) extending concentrically within said discharge pipe (38).

33. An apparatus according to claim 31 or 32, **characterized** in that the cylindrical main part (12) of said reactor drum (10) is rotatably supported by radial roller bearings (14).

34. An apparatus according to anyone of claims 31 to 33, **characterized** in that in said second end wall (28) a stationary central double chamber is provided which is slidably sealed against said rotary end wall (28) and of which a forward flow chamber (76) is connected to said forward flow pipes (72) and a return flow chamber (78) to said return flow pipes (74).

35. An apparatus according to anyone of claims 31 to 34, **characterized** in that said feed pipe (50) and said discharge pipe (38) open into an attached container (42) which is divided by a partition (44) into said charging container which is connected to said feed pipe (50) and designed as said pre-fermenting chamber (46), and an exit chamber (40) connected to said discharge pipe (38).

36. An apparatus according to claim 35, **characterized** in that an exit pipe (64) leaves said exit chamber (40) at a height corresponding to the desired reactor drum filling level.

37. An apparatus according to claim 35 or 36, **characterized** in that said pre-fermenting chamber (46) has at least one charging opening (48) which lies above the vertex of said reactor drum (10).

38. An apparatus according to anyone of the preceding claims, **characterized** in that the wall of said pre-fermenting chamber (46) is provided with heat exchange elements (66', 68) for pre-heating the fresh substrate.

39. An apparatus according to anyone of claims 35 to 38, **characterized** in that the wall of said exit chamber (40) is provided with heat exchange elements (66'') for absorbing the residual heat of the fermented substrate.

## Revendications

1. Appareil pour le production de biogaz à base de substances organiques, comportant un tambour de reaction à axe horizontal avec mélangeur destiné à recevoir ces substances et présentant à l'une de ses extrémités (paroi frontale de chargement) un dispositif d'introduction des substances fraiches et un dispositife de vidange des substances décomposées, et comportant un système de prélèvement du biogaz obtenu, et un système de chauffe pour l'apport de chaleur nécessaire au processus, caractérisé en ce que le tambour de réaction (10) est traversé de façon coaxiale par un tube d'amenée (50) des substances fraiches (62) relié par la paroi frontale de chargement (26) à un bac d'approvisionnement, et dont l'autre extrémité débouche dans le tambour à proximité de la paroi frontale opposée (28), en ce que le tube d'amenée est entouré d'une hélice transporteuse (54) de longueur correspondante à celle du tambour (10), et dont le coté déverseur aboutit vers la paroi frontale de chargement (26) à un tube d'évacuation (38), et en ce que la paroi du tambour (10) est équipée d'un système de chauffe pour l'apport de chaleur nécessaire à la réaction.

2. Appareil selon la revendication 1, caractérisé en ce que l'hélice transporteuse (54) présente en son milieu, au moins sur la plus grande partie de sa longueur, une distance par rapport au tube d'amenée (50), telle qu'une ouverture de passage pratiquement circulaire en vue axiale reste libre entre l'hélice (54) et le tube (50).

3. Appareil selon la revendication 2, caractérisé en ce que l'ouverture de passage (56) vue en coupe longitudinale est délimitée par une surface imaginaire conique, dont la pointe se trouve à proximité de la paroi de chargement (26).

4. Appareil selon une des revendications précédentes, caractérisé en ce que le pas (h) de l'hélice augmente en direction de la seconde paroi frontale (28).

5. Appareil selon une des revendications précédentes, caractérisé en ce qu'il est prévu pour le mouvement de rotation relatif entre tambour et hélice un entrainement rotatif (86) régable dans les deux sens.

6. Appareil selon la revendication 5, caractérisé en ce que l'entrainement rotatif est couplé à un dispositif de réglagle (90) auquel sont reliées des sondes de mesure (92) disposées à l'intérieur du tambour (10) pour la saisie des données de processus.

7. Appareil selon la revendication 5 ou 6, caractérisé en ce que l'entrainement rotatif (86) de l'hélice (54) est fixé dans la partie médiane de la longeur du tambour (10) immobile.

8. Appareil selon la revendication 7, caractérisé en ce que l'entrainement rotatif (86) est enfermé dans un carter qui est étanche par rapport au tambour (10), et d'ou sort l'arbre de transmission (118), muni d'une roue d'entrainement (120) et s'étendant dans une chambre à gaz (116) au sommet du tambour (10).

9. Appareil selon la revendication 8, caratérisé en ce que la roue d'entrainement (120) est un pignon (120) entrainant une courroie crantée (122), elle-meme entrainant la circonférence du tambour.

10. Appareil selon la revendication 8 ou 9, caratérisé en ce que la chambre à gaz est munie d'un tube de sortie (84) pour le prélèvement du biogaz produit.

11. Appareil selon une des revendications précédentes, caratérisé en ce que les substances décomposées sont évacuée par le tube d'évacuation (38) sortant de la paroi frontale de chargement (26), lui-meme relié par une conduite de raccordement (126) à l'entrée du tube d'amenée (56) à l'aide d'une vanne à trois voies (130).

12. Appareil selon la revendication 11, caractérisé en ce que la vanne à trois voies (130) est commandée par un dispositif de régulation relié aux sondes de mesure (132,134) des données de processus pour l'alimentation en substances fraiches.

13. Appareil selon une des revendications précédentes, caractérisé en ce que l'hélice tournante repose sur la paroi intérieure du tambour à l'aide de paliers lisses (98).

14. Appareil selon la revendication 13, caractérisé en ce que chaque palier se compose d'un anneau interne (100) fixé à la périphérie extérieure de l'hélice (54) et d'un anneau externe (102) appuyé de façon rotative à la paroi interne (104) du tambour (10), l'anneau externe (102) s'engrenant avec l'anneau interne (100) pour assurer sa fixation axiale.

15. Appareil selon une des revendications précédentes, caractérisé en ce que l'hélice (54) présente des évidements pour le passage axial du biogaz et des substances.

16. Appareil selon la revendication 15, caractérisé en ce que chaque spire de l'hélice présente un évidement (60) situé dans la zone radialment externe, en ce que tous les évidements (60) sont alignés axialement et en ce que l'entrainement rotatif (86) peut être arreté par un interrupteur de commande tel que les évidements (60) se trouvent en position haute lorsque l'hélice (54) est immobile par rapport au tambour.

17. Appareil selon une des revendications précédentes, caractérisé en ce que des barres axiales de mélange (168) profilées sont fixées aux zones radialement externes des spires de l'hélice (54).

18. Appareil selon une des revendications précédentes, caractérisé en ce que des pales mélangeuses (142) sont fixées à l'hélice (54) pour agiter la substance dans les deux sens de rotation de l'hélice (54).

19. Appareil selon une des revendications précédentes, caractérisé en ce que la seconde paroi frontale (28) du tambour (10) est équipé d'un dispositif supplémentaire d'alimentation (146) en substances fraiches.

20. Appareil selon une des revendications précédentes, caractérisé en ce que la seconde paroi frontale (28) est fixée de façon amovible au tambour (10).

21. Appareil selon une des revendications précédentes, caractérisé en ce que la paroi frontale de chargement (26) du tambour (10) présente une conduite de retour (148) qui sort du tambour (10) au niveau de remplissage et entre dans un bac de chargement faisant fonction de chambre de préfermentation, d'où les substances fraiches sont amenées dans le tube de chargement (50), la section de la conduite de retour (148) étant essentiellement plus petite que celle du tube d'évacuation (38).

22. Appareil selon une des revendications précédentes, caractérisé en ce que la paroi du tambour (10) et/ou du bac de chargement présente une construction en sandwich costituée par une couche médiane isolante (30) et d'une paroi interne lisse (32) en matériau synthétique anticorrosion.

23. Appareil selon une des revendications précédentes, caractérisé en ce que le bac de chargement est divisé par un élément de séparation en une chambre supérieure de fermentation (46) et en un réservoir inférieur d'eau chaude (152).

24. Appareil selon la revendication 23, caractérisé en ce que l'élément de séparation est constitué par une gaine horizontal (158) accessible de l'extérieur et contenant une pompe à substrat (160), dont la sortie est reliée au tube de chargement (50) et dans l'entrée duquel débouche la base d'une trémie (162) non isolée délimitant la base de la chambre de préfermentation (162).

25. Appareil selon la revendication 24, caractérisé en ce que dans le tambour (10) est logé un interrupteur de sécurité qui arrete la pompe (160) lorsque le niveau maximum de remplissage est atteint.

26. Appareil selon une des revendications précédentes, caractérisé en ce que le chauffage intégré dans la paroi du tambour est constitué par échangeurs de chaleur à plaques (66) s'étendant en direction axiale du tambour (10) et disposés sur le périmètre à égale distance les uns des autres.

27. Appareil selon la revendication 26, caractérisé en ce que chaque échangeur (66) présente un tube d'arrivée (72) et un tube de retour (74) pour l'agent caloporteur, en ce que dans la paroi de chargement (26) les tubes d'arrivée (72) débouchent dans au moins une chambre d'arrivée (170), et que les tubes de retour (74) débouchent dans une chambre de retour (176), et en ce que la chambre d'arrivée (170) et la chambre de retour (176) sont reliées au réservoir d'eau chaude (152).

28. Appareil selon la revendication 27, caractérisé en ce que sont prévus deux chambres d'arrivée (170,170') circulaires et concentriques dans lesquelles l'eau chaude circule à contre-courant, chacun des échangeurs de chaleur étant relié alternativement aux tubes d'arrivée (72) de l'autre échangeur de chaleur.

29. Appareil selon une des revendications précédentes, caractérisé en ce que la paroi du tube d'évacuation (38) est équipée d'éléments d'échangeur (140) pour la récupération de la chaleur résiduelle du substrat décomposée vers le réservoir d'eau chaude (152) et/ou vers la chambre de préfermentation.

30. Appareil selon une des revendications précédentes, caractérisé en ce qu'est prévu au moins une pompe de chaleur (150, 156) pour l'alimentation en chaleur du réservoir d'eau chaude (152) et/ou de la chambre de préfermentation (46).

31. Appareil selon l'une des revendications 1 à 6, 15 à 20 et 22 à 30, caractérisé en ce que l'hélice (54) non mobile est fixée à la paroi interne cylindrique (32) du tambour rotatif ainsi qu'au tube de chargement (50).

32. Appareil selon la revendication 31, caractérisé en ce que le tube d'évacuation (38) est monté au centre de la paroi frontale de chargement (26), le tube de chargement (50) passant au centre du tube d'évacuation (38).

33. Appareil selon la revendication 31 ou 32, caractérisé en ce que la partie principale cylindrique (12) du tambour (10) est supportée en rotation sur des coussinets à roulement.

34. Appareil selon une quelconque des revendications 31 à 33, caractérisé en ce qu'au centre de la seconde paroi frontale est prévue une double chambre immobile et à étanchéité glissante par rapport à la paroi rotative (28), et se composant d'une chambre d'arrivée (76) reliée au tubes d'arrivée (72) et d'une chambre de retour (78) reliée au tubes de retour (74).

35. Appareil selon une quelconque des revendications 31 à 34, caractérisé en ce que le tube de chargement (52) et le tube d'évacuation (38) débouchent dans un bac additionnel (42) divisé par une paroi de séparation (44) en un bac de chargement faisant office de chambre de préfermentation (46) et relié au tube de chargement (50) et en une chambre d'évacuation (40) relié au tube d'évacuation (38).

36. Appareil selon la revendication 35, caractérisé en ce que la chambre d'évacuation (40) est reliée à un tube d'évacuation (64) positionné à un niveau correspondant au niveau de remplissage du tambour (10).

37. Appareil selon la revendication 35 ou 36, caractérisé en ce que la chambre de préfermentation (46) présente au moins une ouverture de chargement (48) située plus haut que le sommet du tambour (10).

**38.** Appareil selon une des revendications précédentes, caractérisé en ce que dans la paroi de la chambre de préfermentation (46) sont intégrés des éléments d'échangeur de chaleur (66',68) pour le préchauffage du substrat frais.

**39.** Appareil selon une quelconque des revendications 35 à 38, caractérisé en ce que dans la paroi de la chambre d'évacuation (40) sont intégrés des éléments d'échangeur de chaleur (66'') pour la récupération de la chaleur résiduelle du substrat décomposé.

Fig. 1

Fig. 2

Fig. 3

Fig. 4

Fig. 5

Fig. 6

Fig. 7

EP 0 113 719 B1

Fig. 8

Fig. 19

Fig. 9

EP 0 113 719 B1

Fig. 10

Fig. 11

28

Fig. 12

Fig. 13

Fig. 14

Fig. 15

Fig. 16

Fig. 17

Fig. 18